# FASCICULE DE BREVET EUROPEEN

(11) **EP 1 996 608 B1**
(45) Date de publication et mention de la délivrance du brevet: **15.09.2010**
(21) Numéro de dépôt: 07731176.9
(22) Date de dépôt: 22.03.2007
(51) Int. Cl.: C07K 7/64, C07K 5/12, A61K 38/12

(54) **CYCLOPEPTIDE A ACTIVITE ANTI-CANCEREUSE DERIVE DU COLLAGENE DE TYPE IV**
VON KOLLAGEN TYP IV ABGELEITETES CYCLOPEPTID MIT ANTIKREBSWIRKUNG
CYCLOPEPTIDE WITH ANTI-CANCER ACTIVITY DERIVED FROM COLLAGEN TYPE IV

(30) Priorité: 23.03.2006 FR 0602530
(43) Date de publication de la demande: 03.12.2008
(73) Titulaire: Université De Reims Champagne-Ardenne, 51097 Reims Cedex (FR)
(72) Inventeur: MONBOISSE, Jean-Claude, Paul, Louis, F-51100 Reims (FR); RAMONT, Laurent, Gabriel, Emile, F-51110 Lavannes (FR); BRASSART, Sylvie, Anne, Yvonne, F-51100 Reims (FR); FLOQUET, Nicolas, Joseph, Gilbert, 34725 Saint André de Sangonis (FR); THEVENARD, Jessica, Andrée, Mireille, F-51100 Reims (FR)
(74) Mandataire: Desaix, Anne
(86) Numéro de dépôt international: PCT/FR2007/000487
(87) Numéro de publication internationale: WO 2007/107654

(56) Documents cités:
- WO-A-01/51523
- WO-A-99/62944
- PEROSA F ET AL.: "Generation of biologically active linear and cyclic peptides has revealed a unique fine specificity of rituximab and its possible cross-reactivity with acid sphingomyelinase-like phosphodiesterase 3b precursor" BLOOD, vol. 107, no. 3, 1 février 2006 (2006-02-01), pages 1070-1077, XP002401468
- FLOQUET N ET AL.: "The Antitumor Properties of the .alpha.3(IV)-(185-203) Peptide from the NC1 Domain of Type IV Collagen (Tumstatin) Are Conformation-dependent" THE JOURNAL OF BIOLOGICAL CHEMISTRY, vol. 279, no. 3, 16 janvier 2004 (2004-01-16), pages 2091-2100, XP002401469 cité dans la demande
- SERINI G ET AL: "Integrins and angiogenesis: A sticky business" EXPERIMENTAL CELL RESEARCH, SAN DIEGO, CA, US, vol. 312, no. 5, 10 mars 2006 (2006-03-10), pages 651-658, XP005300764 ISSN: 0014-4827
- THEVENARD J ET AL: "Structural and Antitumor Properties of the YSNSG Cyclopeptide Derived from Tumstatin" CHEMISTRY AND BIOLOGY, CURRENT BIOLOGY, LONDON, GB, vol. 13, no. 12, 26 décembre 2006 (2006-12-26), pages 1307-1315, XP005808721 ISSN: 1074-5521

### SEQUENCE LISTING

<110> Université de Reims Champagne-Ardenne
<120> CYCLOPEPTIDE A ACTIVITE ANTI-CANCEREUSE DERIVE DU COLLAGENE DE TYPE IV
<130> B6598A-AD/LV/KN
<140> New International Patent Application
   <141> 2007-03-22
<150> FR 06/02530
   <151> 2006-03-23
<160> 10
<170> PatentIn version 3.3
<210> 1
   <211> 4
   <212> PRT
   <213> Artificial
<220>
   <223> cyclopeptide
<400> 1
   Tyr Ser Asn Ser
   1
<210> 2
   <211> 5
   <212> PRT
   <213> Artificial
<220>
   <223> Cyclopeptide
<220>
   <221> MIS_FEATURE
   <222> (5)..(5)
   <223> xaa: any amino acid, preferably A, G, S, C, N or T
<400> 2
   Tyr Ser Asn Ser Xaa
   1 5
<210> 3
   <211> 5
   <212> PRT
   <213> Artificial
<220>
   <223> Cyclopeptide
<400> 3
   Tyr Ser Asn Ser Gly
   1 5
<210> 4
   <211> 5
   <212> PRT
   <213> Artificial
<220>
   <223> Cyclopeptide
<400> 4
   Tyr Ser Asn Ser Ala
   1               5
<210> 5
   <211> 7
   <212> PRT
   <213> Artificial
<220>
   <223> Linear heptapeptide
<400> 5
   Cys Asn Tyr Tyr Ser Asn Ser
   1               5
<210> 6
   <211> 19
   <212> PRT
   <213> Artificial
<220>
   <223> NC1 alpha3 (IV) 185-203
<400> 6
   Cys Asn Tyr Tyr Ser Asn Ser Tyr Ser Phe Trp Leu Ala Ser Leu Asn
   1               5                   10                  15
   Pro Glu Arg
<210> 7
   <211> 5
   <212> PRT
   <213> Artificial
<220>
   <223> Cyclopeptide
<220>
   <221> MIS_FEATURE
   <222> (4)..(4)
   <223> Xaa: any amino acid, preferably A, G, S, C, N or T
<400> 7
   Ser Asn Ser Xaa Tyr
   1 5
<210> 8
   <211> 5
   <212> PRT
   <213> Artificial
<220>
   <223> Cyclopeptide
<220>
   <221> MIS_FEATURE
   <222> (3)..(3)
   <223> xaa: any amino acid, preferably A G, S, C, N or T
<400> 8
   Asn Ser Xaa Tyr Ser
   1 5
<210> 9
   <211> 5
   <212> PRT
   <213> Artificial
<220>
   <223> Cyclopeptide
<220>
   <221> MISC_FEATURE
   <222> (2)..(2)
   <223> Xaa: any amino acid, preferably A, G, S, C, N or T
<400> 9
   Ser Xaa Tyr Ser Asn
   1 5
<210> 10
   <211> 5
   <212> PRT
   <213> Artificial
<220>
   <223> Cyclopeptide
<220>
   <221> MISC_FEATURE
   <222> (1)..(1)
   <223> Xaa: any amino acid, preferably A, G, S, C, N or T
<400> 10
   Xaa Tyr Ser Asn Ser
   1               5

## Description

L'invention concerne un cyclopeptide homodétique. C'est-à-dire que le cycle est realisé par la cyclisation des groupements amine et carboxyl des acides aminés N-et C-terminaux, formant ainsi un peptide cyclique lié par une liaison amide caractérisé en ce qu'il comprend la séquence YSNS; ou un cyclopeptide, dont le cycle comprend la séquence YSNS et a un nombre de résidus d'acides aminés compris entre 4 et 6. De façon particulière, le cyclopeptide est un pentapeptide, formant une structure en coude β au niveau des acides aminés YSNS, et susceptible d'être obtenu par formation d'une liaison peptidique entre les deux acides aminés des extrémités du pentapeptide représenté linéairement. Dans un mode de réalisation préféré, le cyclopeptide l'invention est capable de se fixer à l'intégrine αVβ3.

L'utilisation d'un cyclopeptide tel que défini ci-dessus dans le traitement du cancer, et plus particulièrement dans le traitement des différentes formes (ou stades) du mélanome, ainsi que dans la fabrication d'un médicament pour traiter le cancer, font également partie de l'invention. Le cyclopeptide est particulièrement adapté à l'administration d'une composition le contenant, par voie orale.

Le mélanome est le deuxième cancer chez l'Homme en terme de nombre d'années de vie perdues. Durant les dix dernières années, son incidence a augmenté de façon plus importante (3 à 5% par an) que les autres types de cancer, à l'exception du cancer bronchique chez la femme. En l'an 2000, il était estimé qu'un individu sur 100 développerait un mélanome dans le courant de sa vie et qu'un patient sur 2 aurait moins de 50 ans. Ce type de cancer devient inéluctablement un problème majeur de santé publique. Le mélanome est une tumeur maligne de très mauvais pronostic avec un risque élevé de métastases ganglionnaires et viscérales.

Au cours des dernières années, le mécanisme tumoral a été étudié et les mécanismes sous-tendant la progression tumorale peu à peu expliqués. Ainsi, lors de la progression tumorale, les cellules cancéreuses quittent la tumeur primaire, traversent les membranes vasculaires et migrent au sein de la matrice extracellulaire environnante.

L'ensemble de ces phénomènes impliquent la sécrétion puis l'activation d'enzymes protéolytiques, comme les métalloprotéinases matricielles (MMPs) ou le système d'activation du plasminogène (Hornebeck W. et al.). Le profil d'expression de ces enzymes protéolytiques, étudié dans diverses lignées de cellules de mélanome humain ou de souris, révèle une très forte augmentation de l'expression de diverses MMPs, de façon corrélée avec le phénotype invasif de ces cellules (Egeblad M et al.). L'expression à la surface cellulaire de MMP-2, fonctionnellement active, influence directement l'adhésion et l'étalement des cellules sur les composants de la matrice extracellulaire et des membranes basales, et favorise la migration et l'invasion de ces cellules.

L'activation de la MMP-2 requiert la formation de complexes avec son inhibiteur, le TIMP-2, et une autre MMP transmembranaire, la MT1-MMP ou MMP-14 (Egeblad M et al.). Elle requiert également la présence de l'intégrine αVβ3 dont l'expression augmente avec le degré d'invasivité des cellules de mélanome. Cette intégrine sert de récepteur membranaire de la MMP-2, favorise son activation et focalise la forme active de la MMP-2 aux lamellipodes, qui induisent la progression de la cellule cancéreuse au sein de la matrice extracellulaire (Settor REB et al.; Deryugina El et al.). De plus, au cours de la progression tumorale, interviennent de nombreux phénomènes inflammatoires impliquant l'activation de cellules inflammatoires (polynucléaires neutrophiles, monocytes, macrophages,...) qui aboutit à la libération de diverses cytokines et facteurs de croissance, mais aussi de MMPs, comme la MMP-9.

L'augmentation du volume tumoral entraîne une hypoxie et un déficit d'apport des nutriments pour les cellules tumorales. Les effets sont surmontés par l'angiogenèse tumorale, mécanisme de néovascularisation prenant naissance au sein des tumeurs à partir d'un réseau capillaire préexistant. Il est indispensable pour la croissance des tumeurs et le développement des métastases, car il rétablit l'apport d'oxygène et de nutriments. L'activation des cellules endothéliales, induite par l'hypoxie et les protooncogènes au sein des tumeurs, conduit à la dégradation de la membrane basale et de la matrice extracellulaire environnante en mettant en jeu les cascades protéolytiques impliquées dans la progression tumorale. La migration orientée des cellules endothéliales est suivie d'une phase proliférative. Les cellules s'organisent en une structure de type capillaire pour former le réseau vasculaire intratumoral. L'angiogenèse est un facteur pronostique dans divers cancers, dont le mélanome.

Le seul rôle de support mécanique a été longtemps dévolu aux macromolécules de la matrice extracellulaire. Il a été montré, depuis plusieurs années, que certains domaines protéiques constitutifs de ces macromolécules pouvaient également contrôler divers événements physiopathologiques comme la différenciation cellulaire, l'apoptose ou l'expression des gènes. Les matrikines sont des peptides issus de la protéolyse partielle des macromolécules de la matrice extracellulaire capables de réguler l'activité biologique de nombreux types cellulaires (Maquart FX et al.). Les molécules de la matrice extracellulaire, et plus particulièrement les divers constituants des membranes basales (collagène de types IV, XV et XVIII, laminines, protéoglycannes, ...) peuvent réguler l'adhésion et la migration des cellules cancéreuses, par l'intermédiaire de certaines matrikines (Pasco S et al. (2004) ; Ortega N et al.). De même, ces dernières peuvent assurer un contrôle de l'expression et/ou de l'activation des protéases mises en jeu lors de la progression tumorale.

Parmi les macromolécules des membranes basales, le collagène de type IV qui en est le principal constituant, joue un rôle prépondérant dans ce contrôle par l'intermédiaire du domaine non collagénique (NC1) de ses différentes chaînes. Il est constitué par l'association en triple hélice de trois chaînes polypeptidiques α(IV), parmi 6 possibles, α1 (IV) à α6(IV), chacune codée par un gène différent (Kalluri R). L'association la plus fréquente correspond à [α1(IV)2 ; α2(IV)]; elle se rencontre dans l'ensemble des membranes basales. D'autres associations, moins bien déterminées, contiennent les chaînes α3(IV) à α6(IV), dites mineures en raison de leur plus faible expression. La chaîne α3(IV) présente une distribution tissulaire très spécialisée (alvéole pulmonaire, glomérule rénal, capsule du cristallin, ...) (Kalluri R). Des matrikines dérivées des domaines NC1 des chaînes α(IV) du collagène de type IV, ou de leur domaine hélicoïdal, induisent l'adhésion des cellules cancéreuses et contrôlent leurs propriétés invasives (Pasco S et al.(2004) ; Ortega N et al. ; Kalluri R).

Les études précédentes se sont concentrées sur le domaine C-terminal de la chaîne α3(IV) (acides aminés 1438 à 1670 de la séquence NP_000082 ; Numéro d'accession NCBI) appelé domaine NC1 (noncollagenous domain) qui s'est révélé extrêmement intéressant au vu des résultats suivants :
- le domaine NC1 de la chaîne α3(IV) possède une activité inhibitrice sur la prolifération et les propriétés invasives de diverses lignées de cellules cancéreuses. Plus particulièrement, un peptide constitué des acides aminés 185 à 203 du domaine NC1 α3(IV) (CNYYSNSYSFWLASLNPER) peut inhiber la prolifération de ces diverses lignées cellulaires. En revanche, les peptides constitués par les séquences homologues des autres chaînes α(IV) n'ont pas d'effet sur ces mêmes lignées (Han J et al. ; Shahan S et al.).
- par ailleurs, des peptides de séquence [NC1 α3(IV) 185-203] inhibent la migration de cellules de mélanomes ou de fibrosarcome *in vitro.* Cette inhibition de la migration des cellules tumorales semble expliquée par l'effet inhibiteur de la séquence [NC1 α3(IV) 185-203] sur la fixation de la MMP-2 à la membrane plasmique mais également par l'effet inhibiteur sur l'activation de la MMP-2 (Pasco S et al. (2000a)). Des résultats comparables ont été obtenus avec des cellules cancéreuses bronchiques (Martinella-Catusse et al, 2001).
- la séquence [NC1 α3(IV) 185-203] se fixe au complexe intégrine αVβ3-protéine CD47 présent à la surface des cellules de mélanome (Shahan TA et al.). Plus précisément, la séquence [NC1 α3(IV) 185-203] se fixe sur la sous-unité β3 de l'intégrine, indépendamment de CD47 et de la sous-unité α associée (Pasco S et al. (2000b)). De même, le domaine d'interaction entre cette séquence et la sous-unité β3 est indépendant du site de reconnaissance de la séquence RGD, qui sert de site de reconnaissance pour de nombreuses protéines de la matrice extracellulaire sur diverses intégrines.
- le peptide synthétique [NC1 α3(1V) 185-203] inhibe la croissance tumorale de cellules B16F1 de mélanome murin, préincubées avec le peptide synthétique [NC1 α3(1V) 185-203], puis injectées dans des souris syngéniques C57BL6 ; cette inhibition s'exerce par une diminution de la prolifération des cellules de mélanome et de leurs propriétés invasives et par diminution de l'expression de MMP-2 et des activateurs du plasminogène, uPA et tPA. Cet effet inhibiteur dépend de la conformation structurale de la séquence [NC1 α3(1V) 185-203] qui forme un coude β au niveau des acides aminés YSNS (188-191) indispensable à son activité biologique. L'absence de ce coude dans des analogues structuraux abolit l'activité biologique (Floquet et al).
- un peptide de séquence α3(IV) 185-203 est capable d'inhiber l'angiogenèse *in vitro* ainsi qu'*in vivo* comme montré sur des coupes histologiques réalisées dans les tumeurs traitées par ce peptide et au sein desquelles le nombre de vaisseaux sanguins est nettement diminué (Pasco S et al. 2005).
- l'activité inhibitrice la séquence α3(IV) 185-203 sur la croissance tumorale de cellules de mélanomes peut être reproduite par l'utilisation d'un heptapeptide linéaire, contenant les 7 acides aminés N-terminaux CNYYSNS du domaine, incluant la séquence YSNS. Des études structurales ont montré la formation d'une structure en coude β au niveau du tétrapeptide YSNS, indispensable à l'activité biologique. Des peptides homologues ne présentant pas cette conformation particulière sont dépourvus d'activité biologique (Floquet et al).

Malgré la compréhension de ces différents mécanismes mis en oeuvre dans l'initiation et l'évolution des mélanomes, les traitements proposés sont peu nombreux, et surtout limités dans leur efficacité. Ainsi, l'exérèse chirurgicale est actuellement le seul traitement curatif du mélanome de stade 1. Elle doit être précoce et large. Aux stades plus avancés, et notamment après dissémination métastatique, les traitements palliatifs actuels sont décevants et peu efficaces. Ils sont surtout représentés par la polychimiothérapie et ont fait peu de progrès.

La présente invention propose des molécules, et plus particulièrement des molécules élaborées à partir de peptides dérivés du collagène de type IV, ayant des propriétés anti-tumorales, et répondant aux exigences imposées pour leur utilisation en thérapie, à savoir une bonne solubilité et biodisponibilité et une activité biologique efficace.

L'invention concerne un cyclopeptide homodétique. C'est-à-dire que le cycle est realisé par la cyclisation des groupements amine et carboxyl des acides aminés N-et C-terminaux, formant ainsi un peptide cyclique lié par une liaison amide caractérisé en ce qu'il comprend la séquence YSNS; ou un cyclopeptide, dont le cycle comprend la séquence YSNS et a un nombre de résidus d'acides aminés compris entre 4 et 6. Selon un mode de réalisation particulier de l'invention, ce cyclopeptide est capable de se fixer à l'intégrine αVβ3.

Par « cyclopeptide », on entend une molécule formée d'une séquence de résidus d'acides aminés (peptide) pour laquelle il existe au moins une liaison stable entre deux de ces résidus, permettant la formation d'un cycle constitué par l'ensemble des résidus d'acides aminés ou par une partie de la séquence des acides aminés la dite partie comprenant les résidus YSNS consécutifs, soit liés entre eux par des liaisons peptidiques, soit pour au moins deux d'entre eux par la susdite liaison stable. La susdite liaison stable ferme donc le cycle, au niveau de deux résidus de la séquence d'acides aminés. Alternativement, on emploiera également l'expression « peptide cyclique », par opposition aux termes « peptide linéaire » ou « peptide acyclique ».
- Par "cyclopeptide homodétique" on entend un cyclopeptide qui consiste uniquement en des résidus d'acides aminés liés les uns aux autres par des liaisons peptidiques ou liaisons eupeptides (liaison peptidique entre la fonction alpha-carboxyl d'un acide aminé et la fonction alpha-amine d'un autre acide aminé). Par contre, les cyclopeptides hétérodétiques consistent en des résidus d'acides aminés liés les uns aux autres par des liaisons peptidiques et au moins une liaison d'une autre nature telle que les liaison ester, pont disulfure, liaison carbone-carbone, liaison carbone-azote, liaison azote-hydrogène ou liaison carbone-soufre. Dans ce cas, la liaison peut avoir lieu entre les fonctions des acides aminés N- et C- terminaux, entre la fonction d'un acide aminé terminal et la fonction d'une chaîne latérale (acide aminé interne) ou entre deux chaînes latérales.

Dans un mode de réalisation particulier, l'invention concerne un cyclopeptide homodétique comprenant la séquence YSNS.

Les cyclopeptides de l'invention, ou dans un mode de réalisation particulier le cycle de ces cyclopeptides, ont une taille d'au moins 4 acides aminés, et par exemple une taille inférieure à 10 acides aminés, et particulièrement un nombre de résidus d'acides aminés variant de 4 à 6, et plus particulièrement exactement 4 (cyclotetrapeptide), exactement 5 (cyclopentapeptide) ou exactement 6 (cyclohexapeptide).

Dans un mode de réalisation particulier, le cyclopeptide selon l'invention, ne consiste pas en un cyclopeptide de séquence ACPYSNSSLC.

A titre d'exemple, un cyclopentapeptide hétérodétique selon l'invention sera représenté par la formule 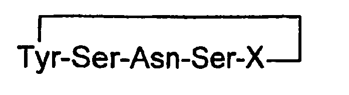 ou 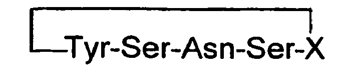 dans laquelle un trait horizontal à coté de l'acide aminé indique une liaison avec la fonction amine ou carboxyle (normalement engagée dans la liaison peptidique), et un trait vertical au-dessus de l'acide aminé indique une liaison avec un autre groupement que ceux indiqués ci-dessus.

A titre d'exemple, un cyclopentapeptide homodétique est représenté par l'une quelconque des formules suivantes :

(I) cyclo(Y-S-N-S-X) ou cyclo(Tyr-Ser-Asn-Ser-X),

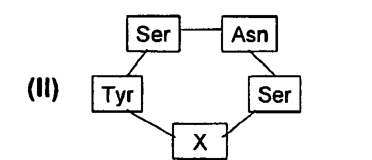

ou

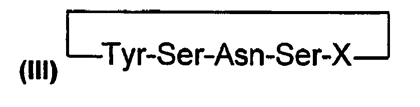

Parmi les propriétés fonctionnelles des cyclopeptides de l'invention illustrant leur utilité dans l'élaboration de compositions antitumorales, dans un mode de réalisation particulier ceux-ci se fixent à l'intégrine αVβ3. Dans un mode de réalisation particulier, les cyclopeptides ont une fixation à l'intégrine αVβ3 au moins aussi efficace que celle du peptide linéaire de séquence [NC1 α3(IV) 185-203] et/ou de l'heptapeptide linéaire CNYYSNS. De façon encore plus particulière, la fixation du cyclopeptide à l'intégrine αVβ3 est plus efficace que celle des peptides linéaires décrits ci-dessus.

Les études de fixation de l'heptapeptide linéaire CNYYSNS ou du cyclopeptide YSNSG de l'invention sont réalisées par compétition avec le peptide natif linéaire [NC1α3(IV) 185-206] marqué à la biotine (Pasco et al, 2000b]. Brièvement, des cellules de mélanome, par exemple des cellules UACC903, HT-144 ou A-375 (10⁶ cellules) sont préincubées *in vitro* pendant 15 min en présence de l'heptapeptide linéaire CNYYSNS (0 à 200 µM) ou du cyclopeptide YSNSG (0 à 200 µM). Les cellules sont lavées 3 fois, puis incubées pendant 30 min avec le peptide [NC1α3(IV) 185-206] marqué à la biotine. Les cellules sont ensuite lavées 3 fois et incubées avec un anticorps monoclonal anti-biotine marqué au FITC (fluorescéine isothiocyanate) dilution 1 :75. Les cellules sont ensuite fixées et analysées en immunofluorescence.

De même, les régions potentielles d'interaction du ou des peptides cycliques de l'invention et/ou leurs versions linéaires sont caractérisées par des méthodes d'amarrage moléculaire (docking). Ces méthodes consistent à prédire le mode de liaison et l'affinité de ligands flexibles, en connaissant la structure de la cible, considérée comme rigide. La structure de l'intégrine αVβ3 a été publiée. Avec le logiciel Autodock, le protocole d'étude de « docking » consiste à lire la structure de la cible (disponible dans Protein Data Bank), puis à tester successivement toutes les positions et orientations possibles du ligand à sa surface. Les positions sont classées selon une fonction de score décrivant l'énergie libre de fixation du ligand à la protéine cible. Les meilleures positions sont analysées en termes de régions de fortes affinités à la surface du récepteur (clusters). Les régions concernées sont ensuite expérimentalement vérifiées en étudiant les interactions entre les peptides de l'invention et des domaines recombinants, mutés pour les acides aminés impliqués, de l'intégrine β3, par la technique utilisant un appareil Biacore.

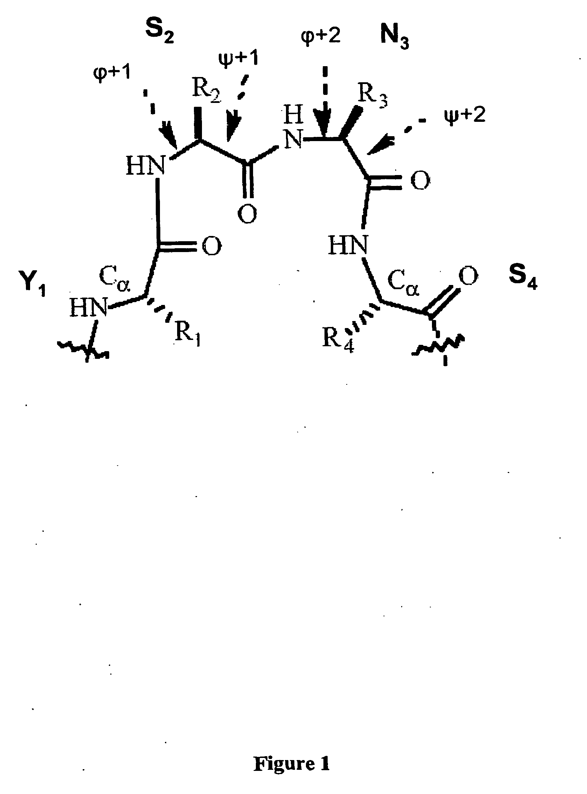

Les cyclopeptides de l'invention, qu'ils soient notamment homodétiques ou hétérodétiques, peuvent également être caractérisés par la présence au niveau des acides aminés YSNS d'une structure en coude β, telle que celle représentée à la Figure 1. De façon particulière, les angles dièdres ϕ et ψ des deux résidus centraux du coude Y(SN)S sont respectivement d'environ -90 et environ -66 degrés. Le coude β correspondant est proche d'un coude β de type I. Ainsi, le coude β formé
par les acides aminés YSNS est du type I, du type VIII (classification selon Hutchinson EG et al.) ou similaire à ces types de coude β de par les angles dièdres susmentionnés. De façon particulière, le cyclopeptide de l'invention présente un coude β ayant une valeur ψ+1 d'environ -60° et une valeur ϕ+2 d'environ -90°. La présence d'un coude β dans un des cyclopeptides de l'invention requiert que les liaisons peptidiques entre les résidus d'acides aminés Y₁ et S₂, S₂ et N₃, et N₃ et S₄ soient dans une orientation trans.

Les cyclopeptides de l'invention ont des capacités anti-tumorales illustrées par le fait qu'au moins l'une des propriétés suivantes est vérifiée : leur capacité à inhiber la prolifération de cellules tumorales, leur capacité à inhiber la migration de cellules tumorales ou une activité anti-angiogénique. Un cyclopeptide est considéré comme ayant au moins une de ces propriétés si l'inhibition précitée de la prolifération ou de la migration cellulaire, ou l'activité anti-angiogénique est au moins aussi efficace que celle enregistrée avec l'heptapeptide linéaire CNYYSNS.

L'inhibition de la prolifération ou de la migration des cellules tumorales peut être testée comme indiqué dans la partie intitulée « méthodes » aux points A.9. et A.10. respectivement. Les expériences décrites à ces paragraphes peuvent être réalisées avec tous types de cellules tumorales, et particulièrement des cellules tumorales de mélanome telles que les cellules UACC-903, les cellules HT-144 (ATCC HTB-63), les cellules A375 (ATCC CRL-1619) ou les cellules G-361 (ATCC CRL-1424) (LGC Promochem-ATCC). Quant à l'activité anti-angiogénique, elle peut être testée selon la méthode décrite au point A.15. sur des cellules endothéliales telles que des cellules HMEC-1 (Ades, Atlanta) ou des cellules HUVEC (Promocell, Heidelberg, Allemagne).

Ainsi, un cyclopeptide selon l'invention est considéré comme efficace au niveau de l'inhibition de la prolifération de cellules tumorales (et plus particulièrement de cellules issues de mélanome) lorsque, en présence dudit cyclopeptide, le pourcentage de prolifération des cellules observé *in* vitro et de préférence *in vivo* est réduit d'au moins 20%, d'au moins 30% ou d'au moins 40%, avec des concentrations en cyclopeptide aussi faibles que de 5 à 20 µM. De façon préférée, une réduction de 50% de la prolifération des cellules tumorales est atteinte avec une concentration de 20 µM pour un nombre initial de 20000 cellules par puits.

Par ailleurs, un cyclopeptide selon l'invention est considéré comme efficace au niveau de l'inhibition de la migration de cellules tumorales (et plus particulièrement de cellules issues de mélanome) lorsque le nombre de cellules observées est réduit d'un facteur d'au moins 1,5 après observation *in vitro* et de préférence *in vivo.* De préférence, une réduction d'un facteur 2 est considérée comme particulièrement efficace à une concentration de 20 µM pour un nombre de cellules initial de 5.10⁴.

Enfin, l'activité anti-angiogénique est évaluée par la capacité des cellules endothéliales (cellules HMEC-1 ou cellules HUVEC) à former des pseudo-tubes capillaires lorsqu'elles sont cultivées sur un gel de Matrigel. Un cyclopeptide selon l'invention est considéré comme ayant une activité anti-angiogénique lorsque le pourcentage du nombre ou de la taille des pseudo-tubes capillaires est réduit d'au moins 40%, de préférence au moins 50%, pour une concentration d'au moins 10 µM de cyclopeptide.

En plus des caractéristiques fonctionnelles ci-dessus, le cyclopeptide de l'invention est suffisamment biodisponible et/ou stable pour être utilisé dans des compositions pharmaceutiques. En particulier, le cyclopeptide est aussi biodisponible et/ou aussi stable que l'heptapeptide linéaire CNYYSNS.

Par « peptide biodisponible », on entend un peptide capable de traverser les différentes barrières biologiques pour atteindre les cellules cibles après son administration, et particulièrement de traverser la barrière intestinale après administration orale. La biodisponibilité est évaluée pour un type cellulaire choisi selon l'application envisagée.

Par « peptide stable », on entend un peptide qui a une durée de vie, une fois administré *in vivo,* suffisante pour atteindre les cellules cibles et exercer son action biologique. Un tel peptide a notamment une conformation lui permettant d'être protégé vis-à-vis de la dégradation par les protéases cellulaires, tout en gardant son activité biologique. Une indication de la stabilité du peptide peut être obtenue par des tests réalisés *in vitro.* La dégradation *in vitro* du cyclopeptide est mesurée par contact avec diverses protéases purifiées, disponibles dans le commerce, pendant des périodes d'incubation de durée croissante (1 heure à 72 heures par exemple). La dégradation du peptide est ensuite mise en évidence par HPLC en phase inverse, en comparant les profils obtenus avant et après digestion. L'activité biologique des peptides soumis à la dégradation protéolytique est vérifiée par mesure de l'inhibition de la migration des cellules tumorales selon la technique décrite.

Dans un mode de réalisation particulier, le cyclopeptide est un tétrapeptide de séquence YSNS, pouvant être représenté par la formule :

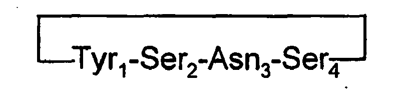

La cyclisation du peptide linéaire YSNS est facilitée par la proximité qui existe entre les groupements Cα (voir Figure 1) de Y₁ et S₄, distants de moins de 7 Å.

Dans un autre mode de réalisation, le cyclopeptide est un cyclopentapeptide de séquence YSNSX de formule telle que définie ci-dessus, et où X est n'importe quel acide aminé permettant la cyclisation du peptide linéaire de même séquence. La nature du résidu d'acide aminé X est contrainte d'une part par la formation d'un cyclopeptide, éventuellement homodétique, et d'autre part par la conservation du coude β au niveau des résidus YSNS. Préférentiellement, on utilisera un acide aminé de très faible volume (tel que l'Alanine, la Glycine ou la Sérine) ou de faible volume (tel que la Cystéine, l'Asparagine ou la Thréonine) pour lequel les chaînes latérales n'empêcheront pas la formation des liaisons peptidiques entre Y₁ et X₅ et entre S₄ et X₅. Alternativement ou en combinaison avec la contrainte rapportée ci-dessus, on utilisera préférentiellement un acide aminé peu chargé ou neutre (tel que l'Alanine, l'Asparagine, la Cysteine, la Glycine, la Sérine et la Thréonine) pour éviter toute interaction indésirable qui altérerait la conformation en coude β. Parmi ces acides aminés, on retiendra tout particulièrement l'Alanine ou la Glycine.

Ainsi, un cyclopeptide de l'invention est un cyclopeptide, homodétique ou hétérodétique consistant en la séquence YSNSG. De façon préférée, le cyclopeptide est un pentacyclopeptide homodétique, pour lequel les 5 acides aminés sont reliés par des liaisons peptidiques, de formule 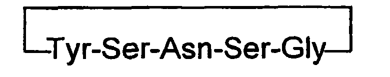 ou de formule

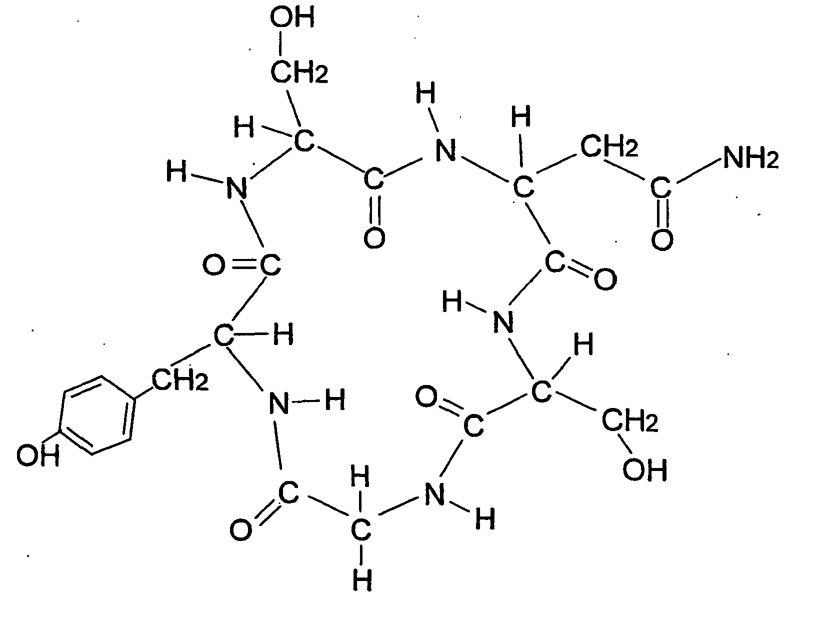

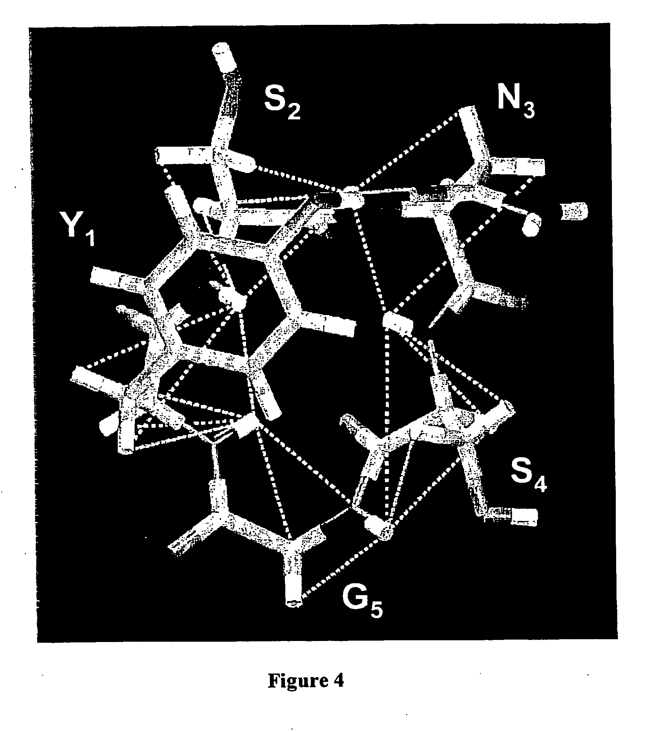

Dans un mode de réalisation de l'invention, le cyclopeptide homodétique (quelle que soit sa taille) possède des liaisons peptidiques en trans. Ainsi, dans un exemple préféré, les 5 liaisons peptidiques du pentacyclopeptide homodétique de séquence YSNSG sont en trans. Il a été montré qu'un tel polypeptide était contraint dans sa conformation β au niveau des acides aminés YSNS du fait des liaisons peptidiques en trans. Un cyclopeptide particulier de l'invention est celui qui possède la conformation tridimensionnelle représentée à la Figure 4, telle qu'obtenue par recherche de conformation aléatoire (voir méthode, point A.5).

Le choix de l'acide aminé ou des acides aminés composant le cyclopeptide, en plus de la séquence YSNS, doit répondre aux contraintes structurales et fonctionnelles indiquées ci-dessus. Parmi les contraintes structurales, l'acide aminé (ou les acides aminés) est préférentiellement peu chargé ou neutre et/ou de faible volume. De plus, le choix de cet ou ces acide(s) aminé(s) doit permettre la formation d'un coude β au niveau des acides aminés YSNS possédant une valeur d'angle dièdre ψ+1 entre -90 et -30° et une valeur d'angle dièdre ϕ+2 entre -120 et -60° (Figure 1). Parmi les contraintes fonctionnelles, le cyclopeptide résultant doit être capable de se lier à l'intégrine αVβ3 et/ou d'inhiber la prolifération de cellules de mélanomes et/ou d'inhiber la migration de cellules de mélanomes et/ou d'inhiber l'angiogénèse. Ainsi, le résidu d'acide aminé glycine (G) peut constituer un choix intéressant du fait de la courte longueur de sa chaîne latérale qui pourrait par conséquent augmenter les chances de cyclisation et diminuer les risques d'obtenir un peptide comportant des liaisons peptidiques en position cis.

Dans un mode de réalisation particulier, le cyclopeptide selon l'invention est susceptible d'être obtenu par cyclisation d'un peptide linéaire comprenant la séquence YSNS, particulièrement un peptide linéaire consistant en 4, 5, 6 ou 7 acides aminés, la séquence cyclique consistant en ou comprenant la séquence peptidique Y-S-N-S. L'obtention du cyclopeptide à partir du peptide linéaire de même séquence est possible par des méthodes classiques de cyclisation. Brièvement, le peptide linéaire est déprotégé sur son extrémité C-terminale, puis N-terminale. L'étape de cyclisation se déroule en phase liquide. Après activation spécifique, la fonction COOH rendue réactive peut subir l'attaque nucléophile de l'azote basique (extrémité N-terminale) pour conduire à la formation de la liaison peptidique recherchée. La réaction de cyclisation est réalisée en haute dilution afin d'éliminer tout risque de dimérisation. L'analyse en HPLC ou en spectroscopie de masse confirme l'aspect monomérique du peptide obtenu (Thern B. et al.).

De préférence, le cyclopeptide est obtenu par formation d'une liaison peptidique entre les acides aminés N- et C-terminaux du peptide linéaire.

Tout particulièrement, un cyclopentapeptide de l'invention est susceptible d'être obtenu par formation d'une liaison peptidique entre les acides aminés Y et X du peptide linéaire YSNSX.

Alternativement, le cyclopeptide peut être obtenu par formation d'une liaison peptidique entre les acides aminés terminaux des peptides linéaires SNSXY, NSXYS, SXYSN et XYSNS (dans lesquels X est défini comme précédemment).

Dans un mode de réalisation particulier, la liaison peptidique formée permettant la cyclisation est réalisée en trans, c'est à dire que les carbones Cα (des deux acides aminés reliés par cette liaison peptidique) se placent de part et d'autre du pont C-N.

Dans un mode de réalisation de l'invention, l'ensemble des liaisons peptidiques est en trans, c'est-à-dire les liaisons peptidiques entre les acides aminés du peptide linéaire, mais également la liaison peptidique permettant la cyclisation.

Dans le cas particulier d'un cyclopentapeptide de séquence YSNSG, la cyclisation est obtenue à partir du peptide linéaire YSNSG, par formation d'une liaison peptidique entre la fonction carboxylique COOH de la glycine (G) et la fonction amine NH₂ libre du résidu N-terminal de tyrosine (Y).

Afin de modifier (accroître ou réduire) sa stabilité ou sa biodisponiblité, le cyclopeptide selon l'invention peut être modifié avant ou après l'étape de cyclisation. Ainsi, l'un quelconque des acides aminés du cyclopeptide peut subir une modification chimique telles que acétylation, alkylation, amidation, carboxylation, hydroxylation ou méthylation, ou peut faire l'objet d'addition de lipides (isoprénylation, palmitoylation, myristoylation ou glypiation) ou de glucides (glycosylation).

Indépendamment ou en combinaison avec les modifications chimiques, le cyclopeptide selon l'invention peut également faire l'objet d'une liaison covalente ou non covalente avec une autre molécule. Ainsi, dans un mode de réalisation particulier le cyclopeptide de l'invention est couplé à un groupement lipidique ou lié (éventuellement de façon covalente) à un transporteur ou à un ligand. Un cyclopeptide lié à une autre molécule sera défini dans le cadre de la présente demande comme un composé hybride.

Toutefois, pour des raisons tenant notamment à la biodisponibilité, le cyclopeptide de l'invention est utilisé sous la forme d'un monomère.

Une composition comprenant au moins un cyclopeptide (ou un composé hybride) décrit ci-dessus fait également partie de l'invention. Ainsi, une composition selon l'invention comprend un seul et unique cyclopeptide, c'est à dire que la composition ne comprend qu'une seule classe de cyclopeptides de taille et séquence identiques et pour lesquels les liaisons peptidiques sont toutes du même type, par exemple les liaisons peptidiques sont toutes en trans.

Une composition particulière selon l'invention comprend un cyclopentapeptide de séquence YSNSG pour lequel l'ensemble des liaisons peptidiques est en trans.

Alternativement, une composition selon l'invention comprend des cyclopeptides pour lesquels la taille et/ou la séquence et/ou la nature des liaisons peptiques est différente, à condition que tous les cyclopeptides comprennent la séquence YSNS. A titre d'exemple, une composition de l'invention peut comprendre des cyclopeptides de même séquence mais pour lesquels la nature des liaisons peptidiques est différente d'un cyclopeptide à un autre, c'est-à-dire soit cis soit trans. Entre également dans le cadre de l'invention une composition qui comprend des cyclopeptides selon l'invention de taille différente par exemple un mélange de tétra, penta et/ou hexacyclopeptides, ou de séquence différente par exemple un mélange de cyclopentapeptides pour lesquels la nature de l'acide aminé X est différente d'un cyclopeptide à un autre (par exemple un mélange de cyclopentapeptides YSNSG et YSNSA).

La composition peut comprendre en outre toute molécule susceptible d'améliorer l'activité biologique du cyclopeptide selon l'invention. Par « améliorer », on entend aussi bien une augmentation de l'activité biologique du cyclopeptide telle que définie ci-dessus dans les tests *in vitro* ou *in vivo,* qu'une meilleure activité biologique au niveau des cellules cibles comparée à une composition comprenant seulement le cyclopeptide ou encore une biodisponibilité ou une stabilité accrue. Ainsi, une molécule capable de favoriser le transport du cyclopeptide vers ses cellules cibles ou capable de diminuer ou retarder (dans le temps) la dégradation du cyclopeptide peut être comprise dans une composition de l'invention. De même, une molécule capable de prolonger l'activité biologique du cyclopeptide peut être incluse dans une composition selon l'invention ; alternativement, le cyclopeptide selon l'invention est inséré dans des nanocapsules, éventuellement biodégradables, permettant ainsi d'améliorer la biodisponibilité, la protection et/ou la délivrance du cyclopeptide.

Fait également partie de l'invention, une composition telle que décrite dans les paragraphes ci-dessus comprenant en outre au moins une molécule d'un autre type, biologiquement active dans le traitement du cancer. Par « molécule biologiquement active dans le traitement du cancer », on entend toute molécule susceptible d'intervenir dans le traitement du cancer, selon la définition donnée ci-après. A titre d'exemple de molécules biologiquement actives dans le traitement du cancer, on peut citer les agents chimiothérapeutiques et les agents immunothérapeutiques classiquement utilisés dans le traitement du mélanome. On citera en particulier la dacarbazine, cisplatine, vindesine, fotémustine ou les nitrosourées. Peuvent également entrer dans la composition selon l'invention, des épitopes tumoraux connus pour être spécifiquement associés aux cellules tumorales et particulièrement ceux associés aux cellules de mélanomes tels que les antigènes Mage 1, 2 et 3, Bage, Gage 1 et 2 ou les antigènes de différenciation mélanocytaire tels que la tyrosinase et Melan-A/MART-1. Enfin, la composition pourra également comprendre l'interleukine 2 ou l'interféron α.

Enfin, lorsque l'une des compositions décrites dans la présente demande est administrée par voie systémique ou locale, particulièrement par injection, la composition comprend en outre un excipient pharmaceutiquement acceptable, ou un transporteur et/ou un véhicule.

La présente demande concerne également une méthode comprenant l'administration *in vivo* d'un cyclopeptide tel que défini ci-dessus ou d'une composition le comprenant pour le traitement de divers types de cancer, plus particulièrement de cellules tumorales exprimant la molécule intégrine αVβ3. Ainsi, un cyclopeptide ou une composition le comprenant peut être utilisé dans le traitement du mélanome ou du cancer bronchique, du cancer du sein ou du cancer de la prostate. Ainsi, fait également partie de l'invention, un cyclopeptide selon l'invention ou une composition le comprenant pour utilisation comme médicament, et plus particulièrement pour utilisation dans le traitement du cancer, tel que le traitement de mélanomes.

Par « traitement du cancer », on entend aussi bien le traitement direct des tumeurs par exemple en réduisant ou stabilisant leur nombre ou leur taille (effet curatif) mais également en évitant la progression *in situ* des cellules tumorales ou leur diffusion, ou l'établissement des tumeurs ; on entend également le traitement des effets néfastes liés à la présence de ces tumeurs, en particulier l'atténuation des symptômes observés chez un patient ou l'amélioration des conditions de vie.

Lorsque différents types de molécules biologiquement actives entrent dans la composition avec les cyclopeptides de l'invention, on peut obtenir un effet synergique contre la tumeur, et en particulier la progression tumorale, c'est-à-dire que la combinaison d'une ou de plusieurs molécule(s) biologiquement active(s) avec un ou plusieurs cyclopeptide(s) selon l'invention a un effet sur la progression tumorale plus important que la somme des effets des molécules et des cyclopeptides utilisés séparément. Les molécules biologiquement actives et les cyclopeptides de l'invention peuvent être administrés ensemble ou séparément dans le temps.

La présente demande concerne également l'utilisation *in vitro* ou *in vivo* d'un cyclopeptide ou d'une composition selon l'invention, dans la diminution de la cascade protéolytique associée à proMMP-2 ou du système d'activation du plasminogène (uPA). L'invention couvre aussi un cyclopeptide ou une composition selon l'invention pour utilisation dans la diminution de la cascade protéolytique associée à proMMP-2 ou du système d'activation du plasminogène (uPA).

Enfin, dans un autre aspect de l'invention, le cyclopeptide ou la composition selon l'invention peut être également être utilisé comme substance anti-angiogénique, aussi bien *in vivo* qu*'in vitro.* Par « substance anti-angiogénique » on entend une substance ayant un effet d'inhibition ou de réduction de la formation de vaisseaux sanguins, de préférence au sein de tumeurs.

Les propriétés anti-angiogéniques d'un cyclopeptide selon l'invention peuvent être par exemple testées comme décrit dans les exemples, sur des cellules HUVEC. On peut notamment rechercher l'effet du cyclopeptide sur la migration cellulaire, par exemple en détectant la sécrétion d'activateurs du plasminogène en présence d'un cyclopeptide de l'invention, ou en dosant certains récepteurs de ces activateurs, ou encore en observant l'effet du cyclopeptide sur la matrice cellulaire.

La présente demande concerne également l'utilisation d'un cyclopeptide ou d'une composition selon l'invention dans la fabrication d'un médicament pour traiter le cancer, et plus particulièrement pour traiter les différents stades de mélanomes. Ainsi, le cyclopeptide selon l'invention peut être utilisé pour traiter des tumeurs primaires, éventuellement avant une exérèse chirurgicale, ou pour traiter les régions environnantes pour limiter le premier stade d'invasion par des métastases cutanées locales.

Le cyclopeptide selon l'invention ou une composition le comprenant est administré(e) à un patient par voie sous-cutanée (s.c.), intradermique (i.d.), intramusculaire (i.m.) ou par injection intraveineuse (i.v.) ou par administration orale. Du fait de sa structure en coude β, le cyclopeptide est protégé vis-à-vis des protéases ce qui lui assure une importante stabilité et biodisponibilité *in vivo.* Par conséquent, le cyclopeptide de l'invention est particulièrement adapté à l'administration par voie orale d'une composition le contenant.

L'invention vise également un kit comprenant une composition telle que décrite dans la présente demande, en particulier une composition formulée pour une administration parentérale, orale, sous-cutanée ou intraveineuse.

### DESCRIPTION DES DESSINS

**Figure 1** : Représentation d'un coude β formé par les acides aminés YSNS et des différents angles dièdres caractéristiques d'un coude β, à savoir ϕ+1, ψ+1, ϕ+2 et ψ+2.

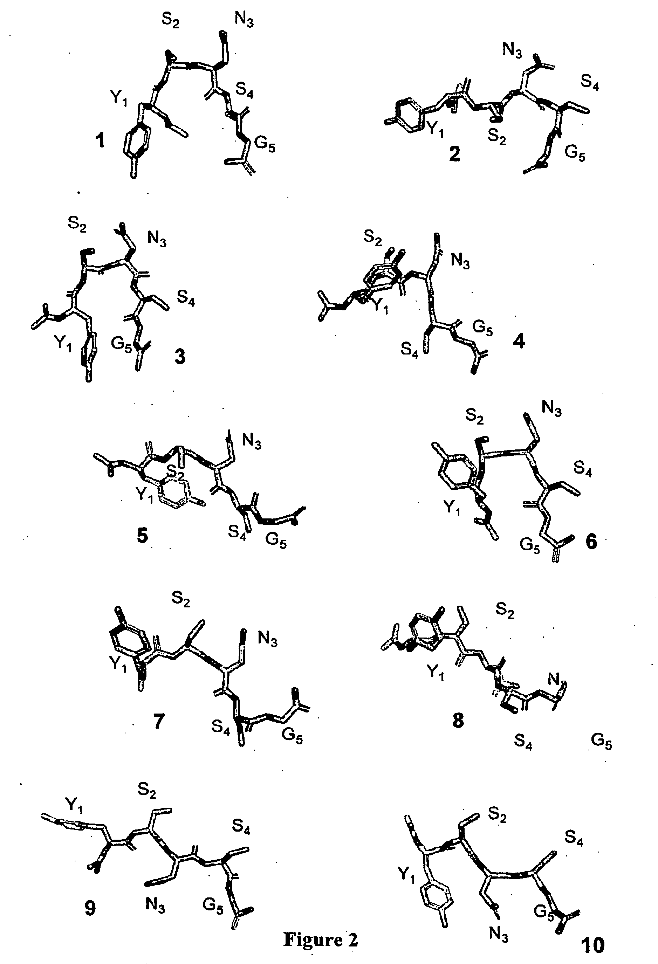

**Figure 2** **:** Représentation des 10 groupes extraits de la simulation dynamique moléculaire du peptide linéaire YSNSG. On distingue 4 classes de structure : une structure en coude β sur les acides aminés YSNS (groupes 1, 3 et 5), une structure en coude β sur les acides aminés SNSG (groupe 2), des structures intermédiaires (groupes 4 et 6), et des structures étendues (groupes 7, 8, 9 et 10). Les acides aminés sont numérotés de la Tyrosine (Y₁) à la Glycine (G₅).

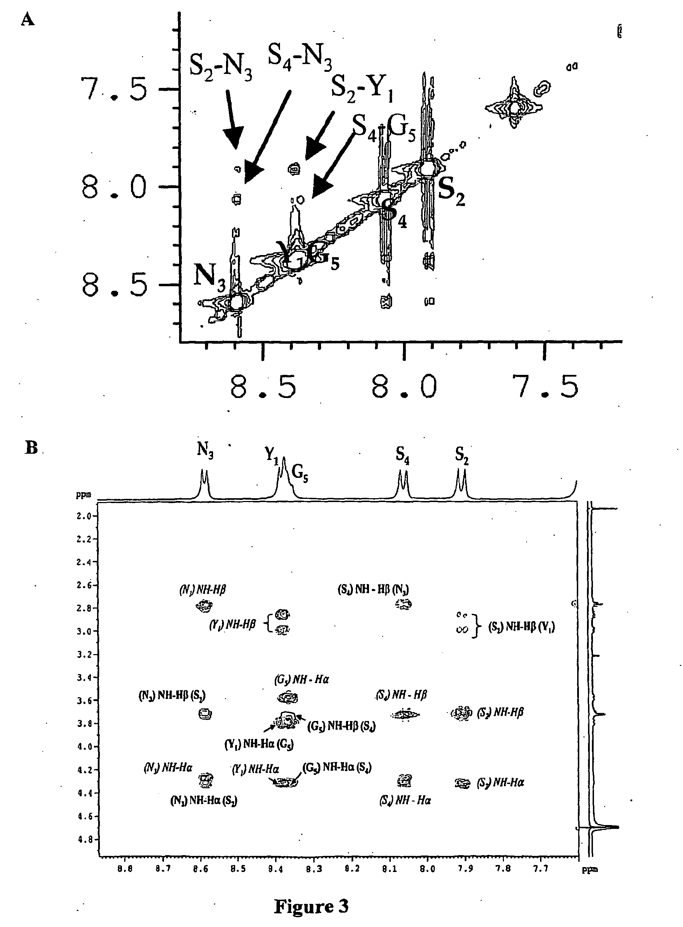

**Figure 3** **:** Résonance magnétique nucléaire du cyclopeptide YSNSG. Parties du spectre ROESY du cyclopeptide YSNSG. Les schémas **A** et **B** décrivent les régions NH-NH et NH-Hα, respectivement. En abscisse et en ordonnée, figurent les valeurs de déplacements chimiques (en ppm : parties pour millions). La position des taches traduit les corrélations entre protons ; ainsi, les protons qui sont à 4 Å au maximum donnent une corrélation sur le spectre RMN 2D.

**Figure 4****:** Conformation d'énergie minimale *in vacuo* du cyclopeptide YSNSG. Cette conformation a été obtenue à partir de la recherche conformationnelle aléatoire *in vacuo* en utilisant une constante diélectrique dépendante de la distance, et réalisée en accord avec les données RMN ; les nOes (effet Overhauser nucléaire) observés expérimentalement qui ont été inclus comme contrainte dans la réalisation de cette conformation sont représentés en ligne pointillée. Cette conformation partage le potentiel d'énergie minimum.

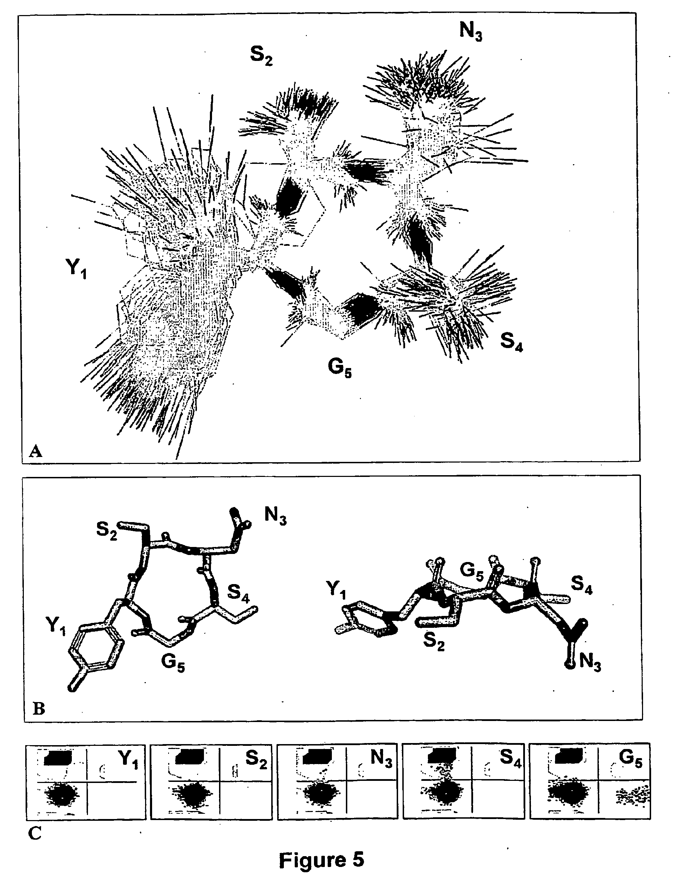

**Figure 5****:** Structure stable du cyclopeptide YSNSG telle qu'observée durant une trajectoire de dynamique moléculaire de 20 ns. La simulation a été réalisée dans une boite d'eau explicite et montre une structure en coude β stable. Le panneau **A** montre que la flexibilité est réduite à la seule chaîne latérale de la tyrosine (Y₁). Le panneau **B** montre que la structure peptidique possède la particularité de présenter une nette séparation des groupements NH des groupements C=O. Enfin, le panneau **C** montre que la particularité de la structure empêche la formation de liaisons hydrogène internes.

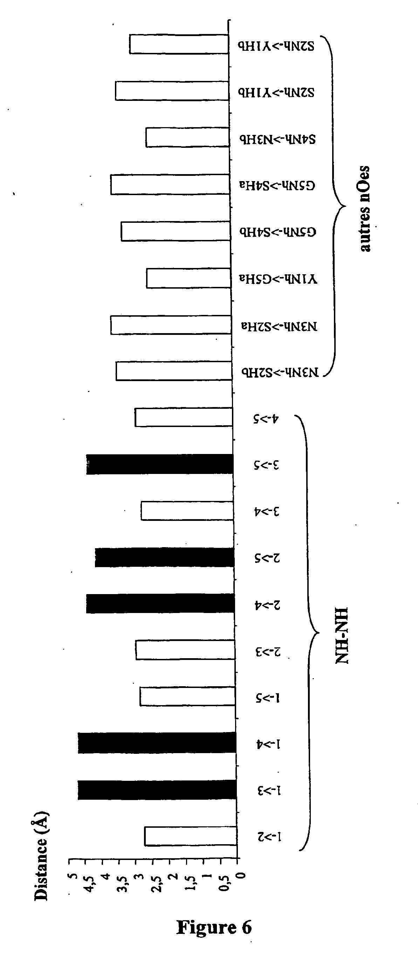

**Figure 6** **:** Distances moyennes proton-proton, extraites de la simulation dynamique moléculaire du cyclopeptide YSNSG. En blanc sont représentés les nOes observés expérimentalement ; en noir les autres nOes. Cette figure montre une bonne corrélation entre les valeurs théoriques et celles observées expérimentalement.

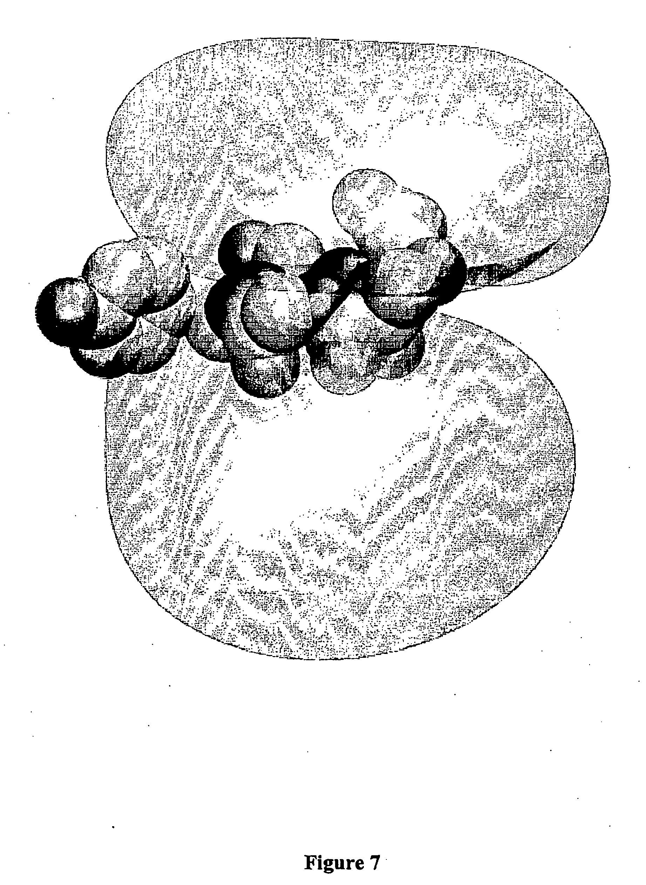

**Figure 7** **:** Potentiel électrostatique du cyclopeptide YSNSG. Les isosurfaces à +7,5 kT/e (surface supérieure) et à -7,5 kT/e (surface inférieure) du cyclopeptide YSNSG (avec T=300 K) ont été calculées et représentées à l'aide de VMD. Cette figure montre l'aspect hautement polarisé du cyclopeptide.

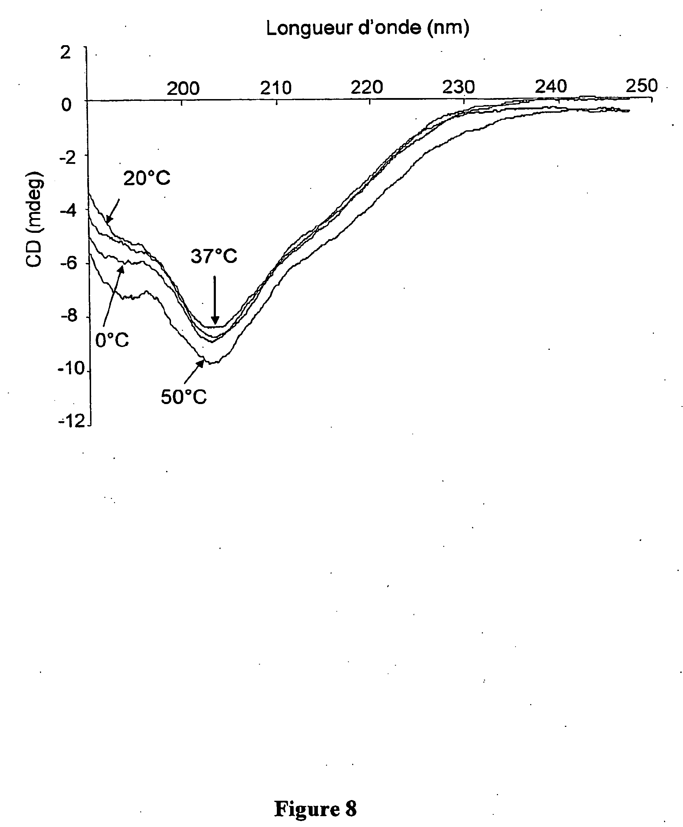

**Figure 8** **:** Spectre de dichroïsme circulaire du cyclopeptide YSNSG. Les différents spectres ont été enregistrés à des températures de 0°C, 20°C, 37°C et 50°C et sont le résultat de 3 accumulations d'un signal dans un intervalle de 190-250 nm.

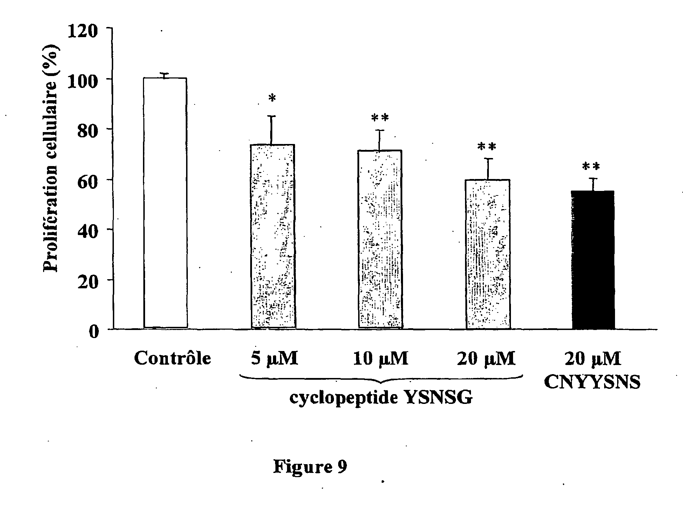

**Figure 9** **:** Test de prolifération *in vitro* des cellules de mélanome humain UACC-903 en présence du cyclopeptide YSNSG. Les cellules UACC-903 ont été incubées pendant 48 heures, dans des plaques de 24 puits, avec un milieu contrôle (témoin négatif, barre blanche), un heptapeptide linéaire CNYYSNS (témoin positif, barre noire) ou des concentrations de cyclopeptide YSNSG variant de 5µM à 20µM (barres grises). La prolifération des cellules a été mesurée avec le réactif Wst-1. * : significativement différent du contrôle (p<0,05) ; ** : significativement différent du contrôle (p<0,01).

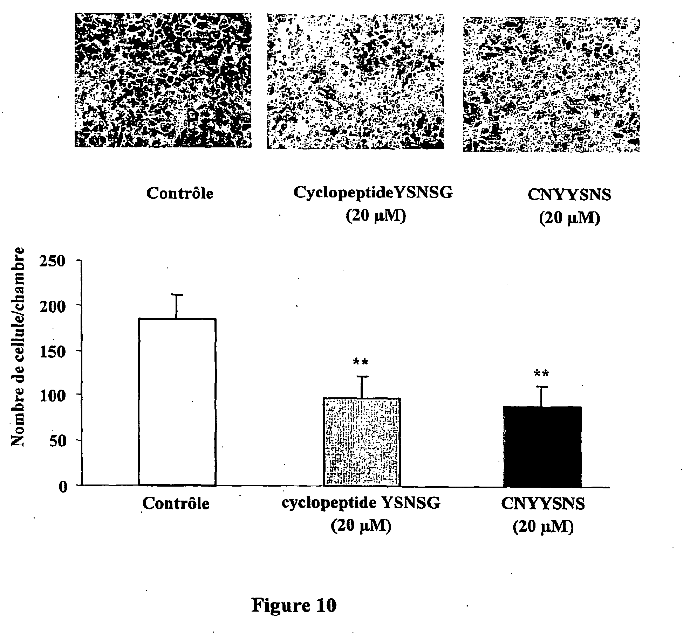

**Figure 10****:** Test de migration *in vitro* des cellules de mélanome humain UACC-903 en présence du cyclopeptide YSNSG. Les cellules UACC-903 ont été incubées avec un milieu contrôle (témoin, négatif, barre blanche), un heptapeptide linéaire CNYYSNS (témoin positif, barre noire) ou avec le cyclopeptide YSNSG à 20µM (barre grise). Après une période d'incubation de 48 heures, les cellules qui ont migré ont été colorées avec du violet cristal et comptées en microscopie inverse. Les résultats ont été exprimés comme la moyenne de deux expériences indépendantes, chacune réalisée en triplicat. ** : significativement différent du contrôle (p<0,01).

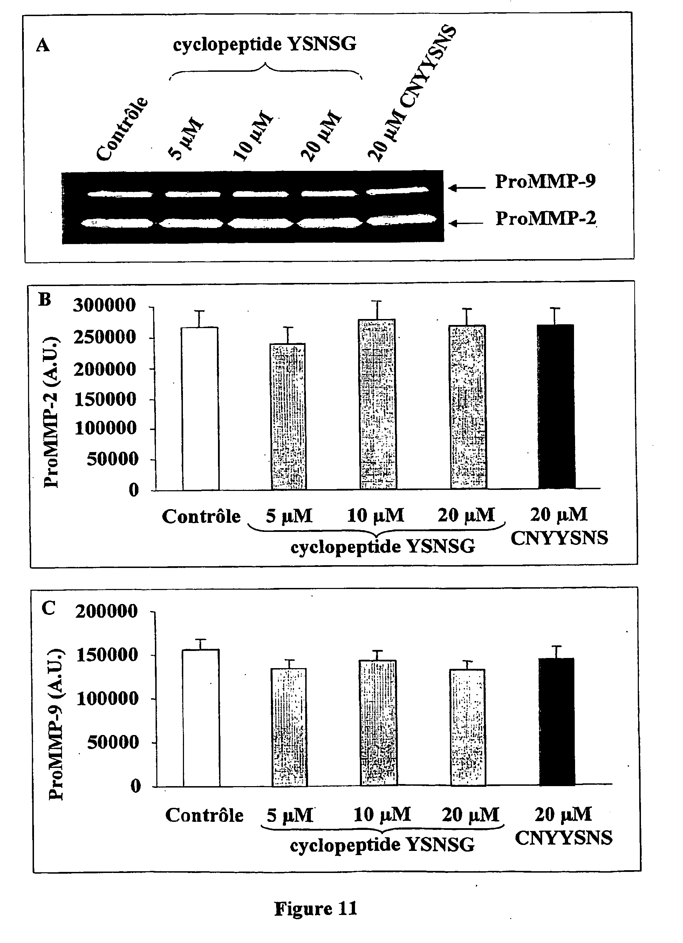

**Figure 11** **:** Effet du cyclopeptide YSNSG sur la sécrétion de MMP-2 et MMP-9. La sécrétion de MMP-2 et MMP-9 a été analysée par zymographie en présence de gélatine **(A),** sur milieu conditionné par des cellules UACC903 en absence de peptide (témoin, négatif, barre blanche), sur des cellules traitées par l'heptapeptide linéaire CNYYSNS (20µM) (témoin positif, barre noire) ou des cellules traitées par des concentrations de 5µM à 20µM de cyclopeptide YSNSG (barres grises). Le milieu conditionné a été recueilli et analysé comme décrit dans les méthodes. La quantification a été effectuée à l'aide du logiciel Bio 1D. **B :** Quantification de ProMMP-2 ; C : Quantification de ProMMP-9. (A.U. : unité arbitraire).

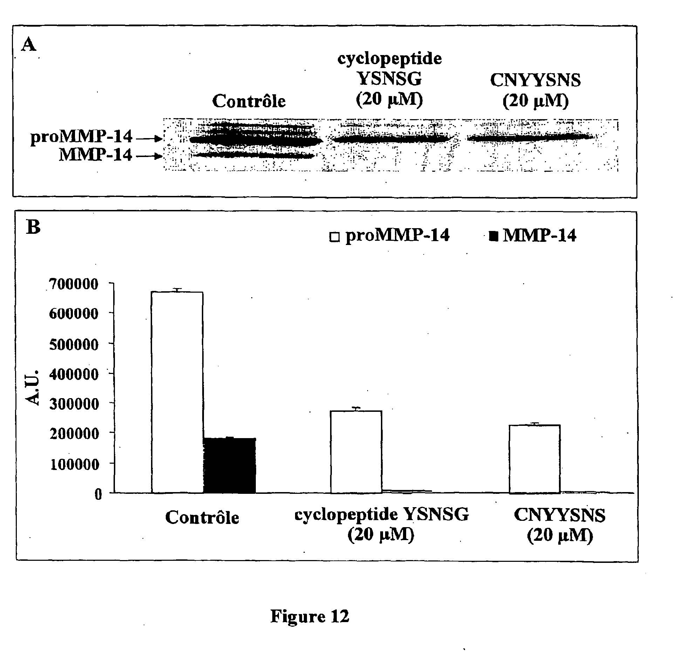

**Figure 12** **:** Effet du cyclopeptide YSNSG sur l'expression et l'activation de MMP-14. L'expression de ProMMP-14 (barre blanche) et MMP14 (barre noire) a été étudiée par Western Blot **(A)** dans un milieu conditionné par des cellules UACC903 cultivées en absence de peptide (contrôle), sur des cellules traitées par l'heptapeptide linéaire CNYYSNS (20µM) (CNYYSNS) ou traitées par le cyclopeptide YSNSG à une concentration de 20µM (cyclopeptide YSNSG). Le milieu conditionné a été recueilli et analysé comme décrit dans les méthodes. La quantification a été effectuée à l'aide du logiciel Bio 1 D **(B).**

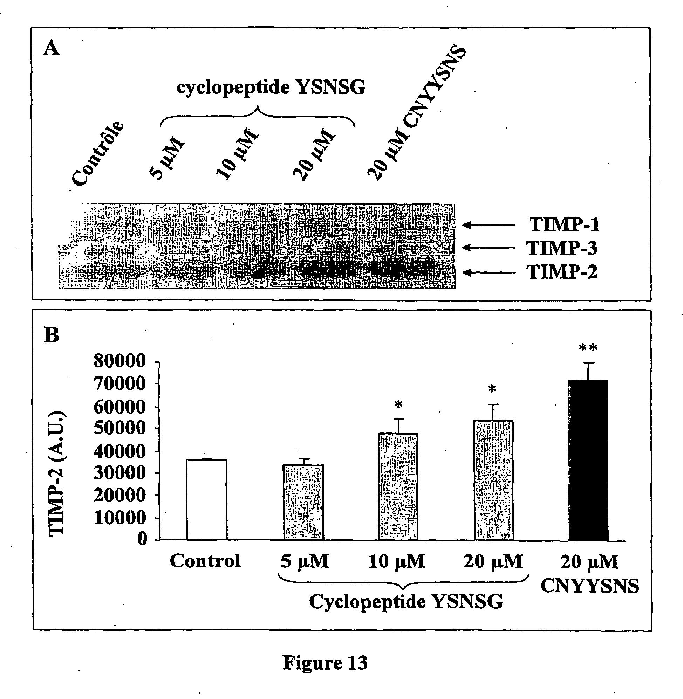

**Figure 13** **:** Effet du cyclopeptide YSNSG sur la sécrétion de TIMP. La sécrétion de 3 inhibiteurs tissulaires de métalloprotéinases ou TIMP (Tissue Inhibitor of MetalloProteinase) a été analysée par zymographie inverse **(A)** dans un milieu conditionné par des cellules UACC903. contrôle en absence de peptide (contrôle négatif, barre blanche), sur des cellules traitées par l'heptapeptide linéaire CNYYSNS (20µM) (contrôle positif, barre noire) ou traitées par le cyclopeptide YSNSG à une concentration variant de 5µM à 20µM (barres grises). Le milieu conditionné a été recueilli et analysé comme décrit dans les méthodes. La quantification de TIMP-2 a été effectuée à l'aide du logiciel Bio 1D **(B).** ** : significativement différent du contrôle (p<0,01).

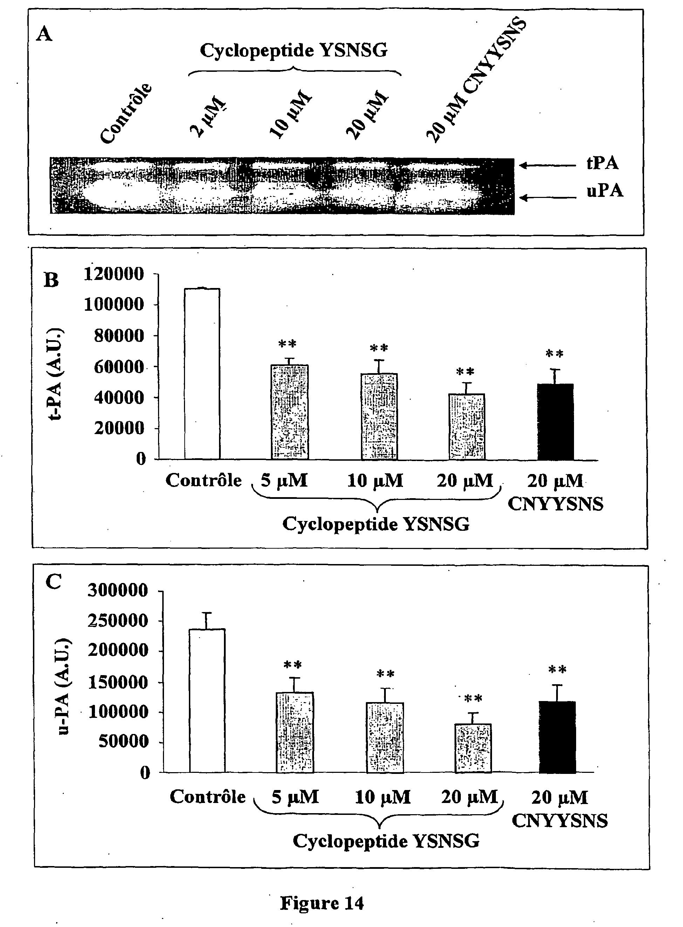

**Figure 14****:** Effet du cyclopeptide YSNSG sur la sécrétion d'activateurs de plasminogène. La sécrétion de u-PA (activateur du plasminogène de type urokinase) et t-PA (activateur tissulaire du plasminogène) a été analysée par zymographie en présence de gélatine et plasminogène **(A)** dans un milieu conditionné par des cellules UACC903 contrôle en absence de peptide (contrôle négatif, barre blanche), sur des cellules traitées par l'heptapeptide linéaire CNYYSNS (20µM) (contrôle positif, barre noire) ou traitées par le cyclopeptide YSNSG à une concentration variant de 5µM à 20µM (barres grises). Le milieu conditionné a été recueilli et analysé comme décrit dans les méthodes. La quantification de t-PA **(B)** et u-PA **(C)** a été effectuée à l'aide du logiciel Bio 1 D. ** : significativement différent du contrôle (p<0,01).

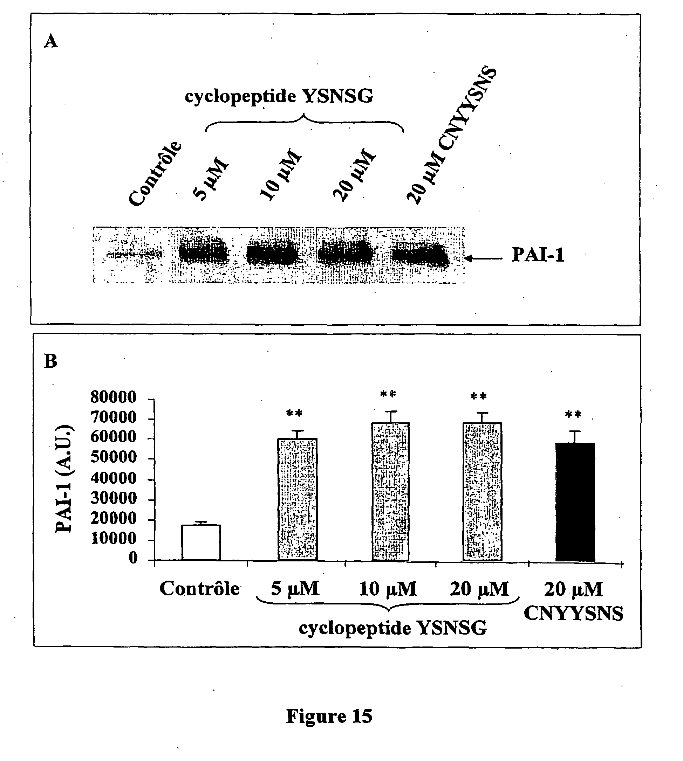

**Figure 15** **:** Effet du cyclopeptide YSNSG sur la sécrétion de PAI-1 (inhibiteur des activateurs du plasminogène 1). La sécrétion de PAI-1 a été analysée par Western Blot **(A)** dans un milieu conditionné par des cellules UACC903 contrôle en absence de peptide (contrôle négatif, barre blanche), sur des cellules traitées par l'heptapeptide linéaire CNYYSNS (20µM) (contrôle positif, barre noire) ou traitées par le cyclopeptide YSNSG à une concentration variant de 5µM à 20µM (barres grises). Le milieu conditionné a été recueilli, concentré et analysé comme décrit dans les méthodes. La quantification de PAI-1 **(B)** a été effectuée à l'aide du logiciel Bio 1D. ** : significativement différent du contrôle (p<0,01).

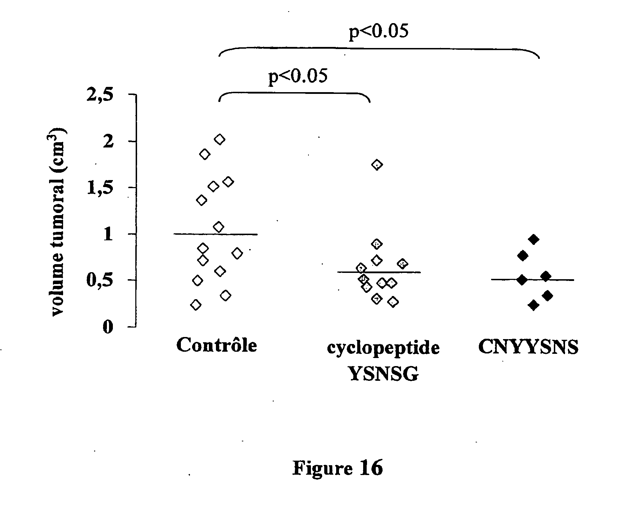

**Figure 16** **:** Test de croissance tumorale *in vivo* en présence du cyclopeptide YSNSG. Des cellules B16F1 ont été incubées pendant 15 minutes avec un milieu contrôle (contrôle négatif, losanges blancs), avec l'heptapeptide linéaire CNYYSNS (20µM) (contrôle positif, losanges noirs) ou avec le cyclopeptide YSNSG à une concentration de 20µM (losanges gris), puis injectées en sous-cutané à des souris syngéniques C57BL6 (2,5.105 cellules par souris). Le volume tumoral a été mesuré au vingtième jour.

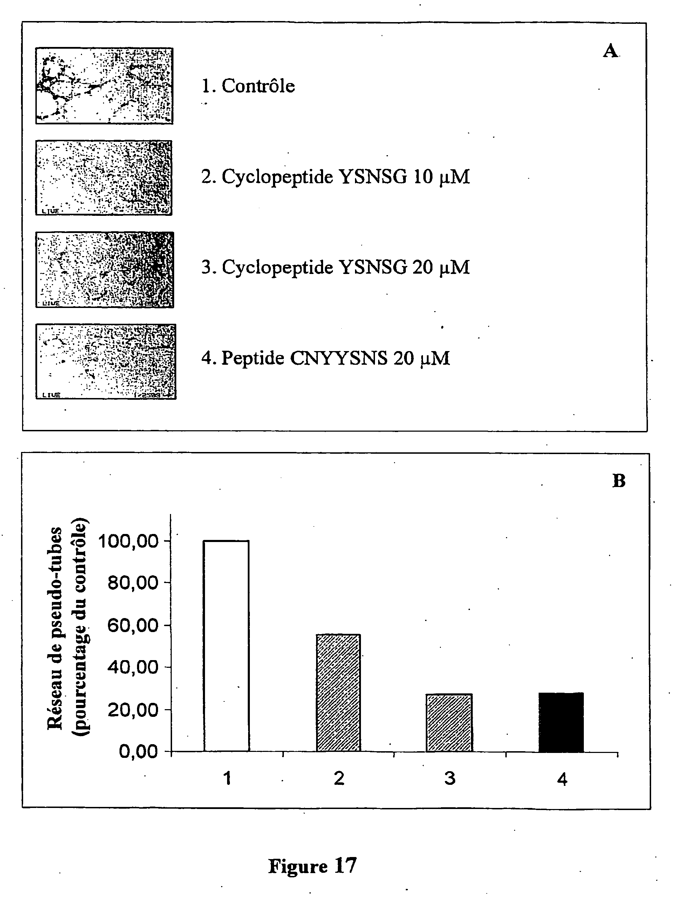

**Figure 17** **:** Activité anti-angiogénique *in vitro* du cyclopeptide YSNSG. **A :** photographies au microscope de réseau de pseudotubes capillaires de cellules endothéliales HMEC-1 après 24 heures d'incubation en l'absence de peptide (contrôle ; 1), avec le cyclopeptide YSNSG à une concentration de 10 ou 20µM (2 et 3 respectivement) ou avec l'heptapeptide linéaire CNYYSNS (20µM) (contrôle positif ; 4). **B :** Quantification du nombre de pseudotubes des photographies en A.

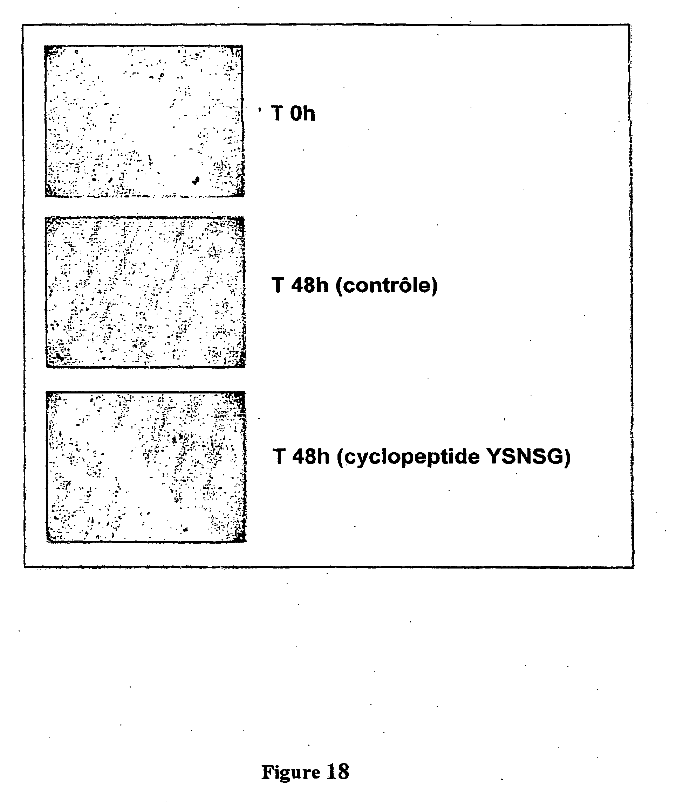

**Figure 18****:** Inhibition *in vitro* de la migration de cellules HUVEC en présence du cyclopeptide YSNSG. Photographies au microscope de blessures artificielles réalisées sur monocouche de cellules endothéliales à T0 et à T48h en absence de peptide (contrôle) ou en présence de 20 µM de cyclopeptide YSNSG.

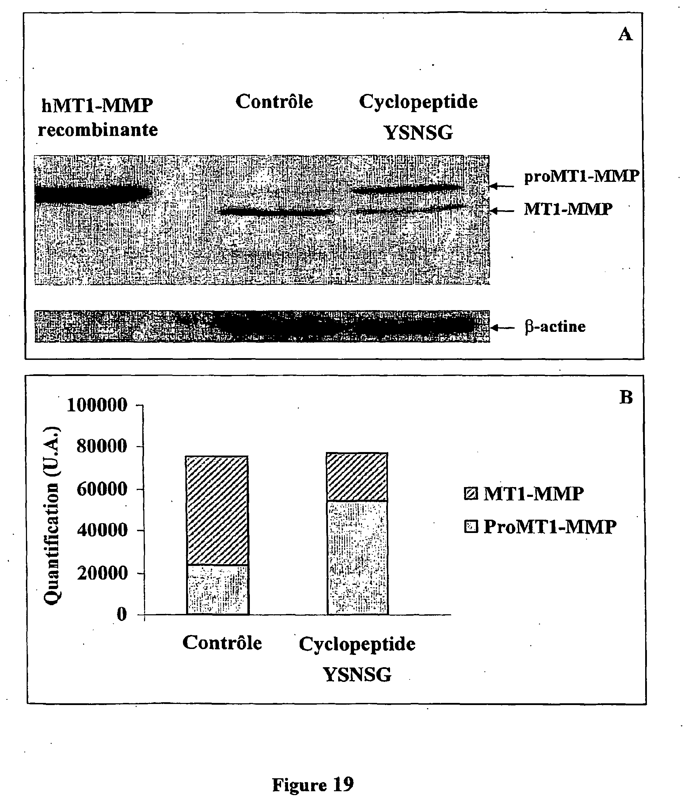

**Figure 19** **:** Effet du cyclopeptide YSNSG sur l'expression et l'activation de MMP-14 par les cellules HUVEC. L'expression de ProMMP-14 et MMP-14 a été étudiée par Western Blot **(A).** Les cellules HUVEC ont été cultivées en absence (contrôle) ou en présence de 20 µM de cyclopeptide YSNSG et les extraits membranaires correspondants ont été analysés. La quantification a été effectuée à l'aide du logiciel Bio 1 D **(B).**

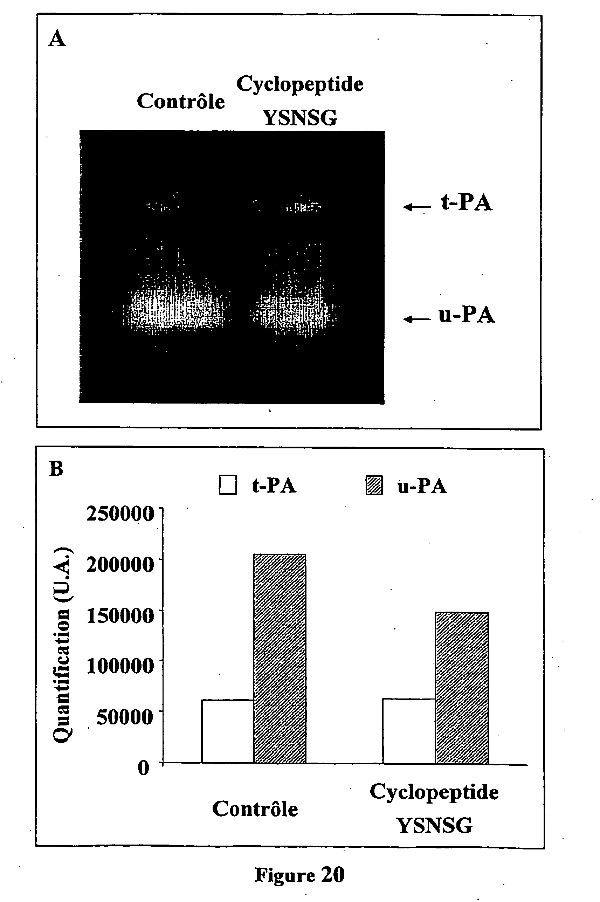

**Figure 20** **:** Effet du cyclopeptide YSNSG sur la sécrétion d'activateurs du plasminogène. La sécrétion d'u-PA et de t-PA a été analysée par zymographie en présence de gélatine et de plasminogène **(A)** dans un milieu conditionné par des cellules HUVEC en absence (contrôle) ou en présence de 20 µM de cyclopeptide YSNSG. La quantification a été effectuée à l'aide du logiciel Bio 1 D **(B).**

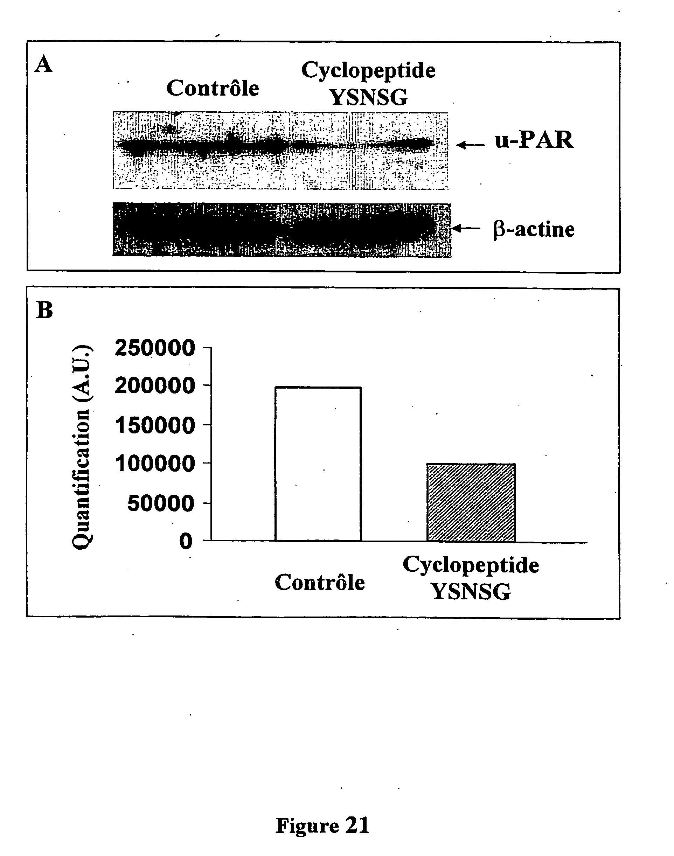

**Figure 21** **:** Effet du cyclopeptide YSNSG sur l'expression du récepteur de l'u-PA : u- PAR par les cellules HUVEC. La sécrétion d'u-PAR a été analysée par Western Blot **(A)** dans un milieu conditionné par des cellules HUVEC en absence (contrôle) ou en présence de 20 µM de cyclopeptide YSNSG. La quantification a été effectuée à l'aide du logiciel Bio 1 D **(B).**

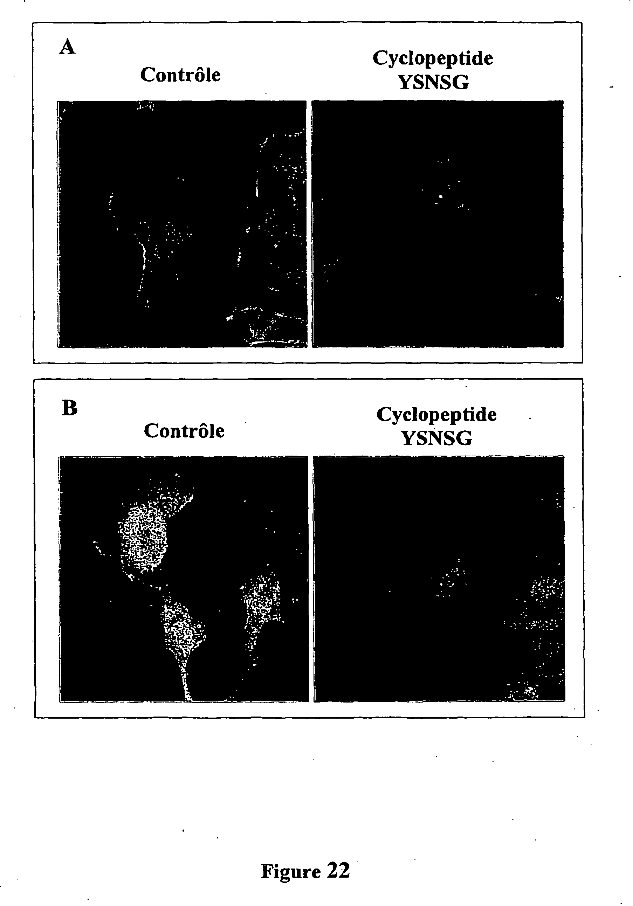

**Figure 22** **:** Effet du cyclopeptide YSNSG sur l'organisation du cytosquelette des cellules HUVEC. Les cellules sont cultivées sur lamelles de verre en absence (contrôle) ou en présence de 20 µM de cyclopeptide YSNSG. Après 48 h d'incubation, les cellules sont fixées, perméabilisées, incubées avec la phalloïdine (A) ou avec un anticorps Anti-Phospho-FAK (B) puis avec du Hoechst-33342. Les lamelles sont observées à l'aide d'un microscope confocal.

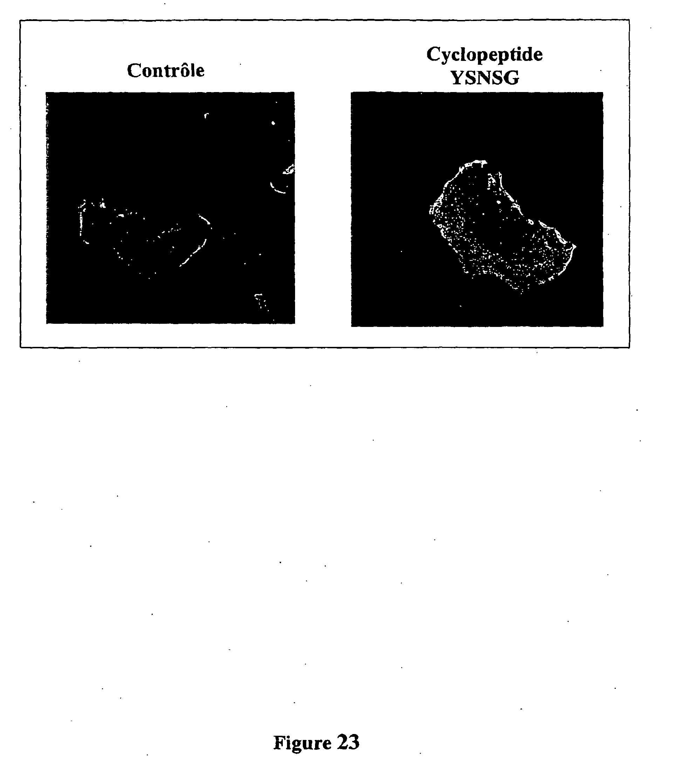

**Figure 23** : Effet du cyclopeptide YSNSG sur la répartition des sous-unités β1 d'intégrine à la surface des cellules HUVEC. Les cellules sont cultivées sur lamelles de verre en absence (contrôle) ou en présence de 20 µM de cyclopeptide YSNSG. Après 48 h d'incubation, les cellules sont fixées et incubées en présence d'anticorps dirigés contre la sous-unité β1 d'intégrine. Les lamelles sont observées à l'aide d'un microscope confocal.

EXEMPLES

**A. Méthodes**

A.1. Réactifs

L'ensemble des réactifs de culture cellulaire et de biologie moléculaire provient de Invitrogen (Cergy Pontoise, France); La sérum albumine bovine (SAB), la gélatine et le Matrigel (ECM gel) proviennent de Sigma (Saint Quentin Fallavier, France). Le plasminogène humain provient de Calbiochem (VWR Int. Strasbourg, France) ; L'anticorps anti-MMP-14 humaine provient de Santa-Cruz Biotech (Tébubio, Le Perray en Yvelines, France). L'anticorps anti-PAI-1 humain provient de American Diagnostica (Neuville sur Oise, France). L'ensemble des réactifs utilisés dans les expériences décrites dans la présente demande est repris dans le tableau 1.

A.2. Peptides

Le peptide linéaire NC1 [α3(IV)185-191] de séquence CNYYSNS a été obtenu par synthèse en phase solide, en utilisant une procédure dérivée de la synthèse FMOC ; il a ensuite été purifié par HPLC en phase inverse avec une colonne C18 par élution sur un gradient d'acétonitrile dans de l'acide trifluoroacétique, puis lyophilisé (Floquet et al.). Le cyclopeptide YSNSG a été commandé auprès de Ansynth Service B.V. (Roosendaal, Netherlands). Le cyclopeptide CYSNG est sous forme acétate et sa pureté est supérieure à 95%, déterminée par HPLC en phase inverse.

A.3. Résonance magnétique nucléaire (RMN)

Les spectres de RMN ont été enregistrés sur un spectromètre Bruker DRX 500. Les échantillons ont été dissous dans un mélange de D₂O : H₂O dans un rapport 9 :1. Le TSP-d₄ (Trimethylsilyl propanoic acid, sodium salt) a été utilisé comme référence interne de déplacement chimique. Le signal de l'eau a été supprimé par la séquence WATERGATE (Piotto M. et al.). La dépendance du déplacement chimique des protons amides a été étudiée à 294 K. L'identification des systèmes de spins a été réalisée par analyse du spectre TOCSY (temps de mélange de 200 ms ; séquence d'impulsion mlevdpst19). La classification des distances entre protons a été réalisée à partir du spectre NOESY (temps de mélange de 350 ms, séquence d'impulsion noesyqpst19). La matrice d'acquisition de ce dernier contient 512 x2K points, étendus à 1Kx 4K par ajout de zéros. La fonction d'apodisation cos² est appliquée dans les deux dimensions avant transformation de Fourier 2D.

**Tableau 1 : Fournisseurs et références des réactifs utilisés**

| **Produits de culture cellulaire** | **Fournisseur** | **Référence** |
|---|---|---|
| Milieu RPMI 1640 | Invitrogen, Cergy-Pontoise, France | 61870-010 |
| Milieu DMEM 4,5 g/l glucose | | 31966-021 |
| Trypsine / EDTA | | 15400-054 |
| Phosphate Buffered Saline | | 10010015 |
| Sérum de veau foetal | | 10270106 |
| Milieu de cultures pour cellules endothéliales ECGM MV | Promocell, Heidelberg, Allemagne | C-22020 |
| Réactif Wst-1 | Roche Diagnostics, Meylan, France | 11644807001 |

| **Produits chimiques** | **Fournisseur** | **Référence** |
|---|---|---|
| Sérum albumine bovine | Sigma, St Quentin Fallavier, France | A 9543 |
| Gélatine | | G8150 |
| Matrigel (ECM gel) | | E1270 |
| Membrane de transfert Immobilon -PVDF | | P2938 |
| Violet crystal | Aldrich, St Quentin Fallavier, France | 86,099-9 |
| Plasminogène EACA | Calbiochem, VWR, Strasbourg, France | 528178.50 |
| Pro-MMP-2 | Oncogene, VWR, Strasbourg, France | PF037 |
| TSP-d4 | Aldrich, St Quentin Fallavier, France | 18,033-5 |

| **Anticorps** | **Fournisseur** | **Référence** |
|---|---|---|
| Anti-MMP-14 | Chemicon, Euromedex, Souffelweyersheim, France | AB815 |
| Anti-PAI-1 | American Diagnostica, Neuviile sur Oise, France | 395G |

| **Matériel de culture cellulaire** | **Fournisseur** | **Référence** |
|---|---|---|
| Plaques 24 puits | Nunc, Dutscher, Brumath, France | 055429 |
| Flacons 25 cm² | Nunc, Dutscher, Brumath, France | 055401 |
| Thincert 8µm | Greiner, Dutscher, Brumath, France | 662638 |

| **Animaux de laboratoires** | **Fournisseur** | **Référence** |
|---|---|---|
| Souris C57BL6 | Harlan, Gannat, France | |

| **Peptides synthétiques** | **Fournisseur** | **Référence** |
|---|---|---|
| Cyclopeptide YSNSG | Ansynth Service, Roosendaal, Pays Bas | |

| **Coffrets de détection** | **Fournisseur** | **Référence** |
|---|---|---|
| ECL Chemiluminescence | Amersham, Saclay, France | RPN2132 |
| Biotrak ELISA TIMP-2 | | RPN 2618 |
| Hyperfilm MP | | RPN3103K |

A.4. Dichroïsme circulaire (CD)

Les spectres de dichroïsme circulaire du cyclopeptide YSNSG ont été enregistrés sur un spectropolarimètre JASCO J-810 CD équipé d'un système de contrôle de température Peltier PTC-623S, en utilisant une cellule de longueur de trajet optique de 0,2 mm. Le peptide est dissous à une concentration de 0,2 mg/ml soit dans de l'eau (pH ~3) ou dans un tampon phosphate de potassium à 20 mM de façon à ce que la solution atteigne un pH ~ 7,3. Les balayages sont acquis dans l'intervalle de spectre 190-250 nm à une température de 0, 20, 37 et 50 °C, en mesurant les points tous les 0,1 nm.

A.5. Recherche conformationelle aléatoire

Partant d'une conformation aléatoire du cyclopeptide YSNSG, une recherche conformationnelle de sa structure a été réalisée in vacuo (isolément) avec une constante diélectrique dépendante de la distance en utilisant le programme CHARMM et un champ de force (PARAM set 22) (Brooks et al. ; MacKerell et al.) en accord avec les données de RMN ; les distances interprotoniques correspondant aux effets Overhauser nucléaires (nOes) observés expérimentalement ont été contraintes à un intervalle de 2,0 à 4,0 Å. De plus, les protons amides du squelette peptidique qui n'étaient pas en vis-à-vis ont été contraints à un intervalle de 4,0 à 8,0 Å. Puisqu'il n'était pas observé de corrélations Hα-Hα sur le spectre de ROESY (données non montrées), toutes les liaisons peptidiques ont été également contraintes dans leur configuration trans (ω ~ 180°). Finalement et en accord avec la relation de Karplus modifiée par Pardi et al., les angles ϕ des deux résidus Sérine (S₂ et S₄) ont été contraints à environ 120° ± 30°, selon les valeurs de couplage constantes ³J_{(NH-Hα)} de 8,2 et 8,8 Hz respectivement (les autres résidus partageant des valeurs entre 5,0 et 7,0 Hz).

A chaque étape du protocole de recherche conformationnelle aléatoire, tous les angles dièdres du squelette peptidique ont été répartis au hasard ; de plus, les structures correspondantes selon les contraintes de l'analyse de RMN ci-dessus ont été minimisées. Chaque modèle généré a été minimisé en utilisant à la fois l'algorithme SD (Steepest Descent) et l'algorithme ABNR (Adopted Based Newton Raphson), jusqu'à ce qu'un gradient d'énergie de 10⁻⁵ kcal.mol⁻¹ ait été atteint. Trois séries indépendantes de 1000 étapes ont été réalisées, en modifiant la graine aléatoire initiale du générateur de nombre aléatoire. Les 3000 structures minimisées générées ont été groupées pour analyse.

A.6. Simulation dynamique moléculaire

Le peptide YSNSG (sous forme linéaire et cyclique) a également été étudié par simulation dynamique moléculaire. Pour le peptide linéaire, le point de départ des calculs fut une conformation entièrement étendue. Pour le cyclopeptide, la simulation a commencé à partir de la conformation demandant le minimum d'énergie, qui a été obtenue précédemment par la recherche conformationnelle aléatoire. Les peptides ont dans un premier temps été incorporés dans une boite d'eau assurant une couche d'eau de 8Å (TIP3P) sur chacun de leur coté, et l'ensemble des systèmes a été minimisé par 2000 étapes de l'algorithme de gradient conjugué et amené à température ambiante (300 K) en utilisant le programme NAMD (Phillips et al. ; Kale et al.). Les deux simulations (sur peptide linéaire ou cyclique) ont été réalisées dans un ensemble NPT (1 atm, 300K) en utilisant l'algorithme de Verlet et une étape d'intégration de 1.0⁻¹⁵ s. Les interactions électrostatiques ont été traitées en appliquant une fonction de lissage au potentiel classique entre 10 et 12 Å (valeur de seuil). Une analyse conformationnelle a été réalisée sur la totalité de la simulation qui atteint 20 ns, en extrayant la structure des peptides tous les 10 ps. Ces modèles ont été regroupés dans un nombre réduit de groupes en utilisant le logiciel NMRCLUST (Kelley et al.) et un seuil de 2,5 Å pour la valeur de seuil du groupe.

A.7. Animaux

Des souris femelles C57BL6 (poids corporel moyen de 18 à 20 g) ont été achetées auprès de Harlan France (Gannat, France). Les animaux ont été placés dans des cages individuelles, à température et humidité maintenues constantes ; la nourriture et l'eau ont été fournies ad libitum. Toutes les souris ont subi une période d'acclimatation de 1 semaine avant le début des expériences. Les expériences *in vivo* ont été réalisées selon les recommandations du Centre National de la Recherche Scientifique (CNRS).

A.B. Culture cellulaire et conditions de culture

Des cellules B16F1, une sous lignée métastasique de cellules pulmonaires de mélanome murin B16 (Dr. M. Grégoire, INSERM U419, Nantes, France) ont été cultivées dans du milieu RPMI 1640, additionné de sérum de veau foetal (SVF) à 5% dans des flacons de 25 cm² (Nunclon, VWR Int, Strasbourg, France) sous atmosphère humidifiée contenant 5% de CO₂. Des cellules UACC-903, une lignée cellulaire de mélanome (Dr J. Trent, Phoenix, Arizona) ont été cultivées dans du milieu DMEM, contenant du glucose 4,5 g/L, additionné de SVF à 5% dans des flacons de 25 cm² (Nunclon) sous atmosphère humidifiée contenant 5% de CO₂. Des cellules HMEC-1 (cellules endothéliales microvasculaires humaines ; E.W. Ades, Center for Disease Control and Prevention, Atlanta, Georgie) et des cellules HUVEC (cellules endothéliales de veines ombilicales humaines; Promocell, Heidelberg, Allemagne) ont été cultivées dans un milieu de croissance de cellules endothéliales (ECGM) additionné de ECGS/H à 0,4% (en poids), de sérum de veau foetal à 2% (en volume), de facteur de croissance épidermique (EGF) à 10 ng/ml, d'hydrocortisone à 1 µg/ml, d'amphotéricine B à 50 ng/ml et de gentamicine à 50 µg/ml.

A.9. Test de prolifération *in vitro*

La prolifération cellulaire a été déterminée en utilisant le réactif Wst-1 selon les instructions du fabricant. Ce réactif contient un sel de tétrazolium qui est réduit par les oxydo-réductases mitochondriales et permet d'évaluer la prolifération et la viabilité des cellules. Sa réduction donne naissance à un dérivé formazan soluble, de coloration jaune. Avant d'atteindre la confluence, les cellules ont été lavées deux fois avec une solution saline tamponnée au phosphate (phosphate buffered saline ; PBS) pour enlever les résidus de SVF et incubées pendant 48 heures dans du milieu DMEM, en présence ou en absence de peptides synthétiques à des concentrations de 0 à 20 µM. Le milieu a été récupéré puis centrifugé à 500g pendant 10 min à 4°C, pour enlever les débris cellulaires. Le contenu protéique du milieu ainsi obtenu est déterminé par la méthode de Bradford, en utilisant la SAB comme étalon.

A.10. Test d'invasion *in vitro*

L'invasion cellulaire a été testée dans des chambres de Boyden modifiées (ThinCert®, insert de culture cellulaire pour plaque de 24 puits, diamètre intérieur de 8,36 mm, pore de 8 µm, Greiner Bio-one, Courtaboeuf France). Brièvement, 5.10⁴ cellules ont été mises en suspension dans du milieu DMEM sans SVF et contenant de la SAB à 0,2% et ensemencées sur des membranes tapissées de matrigel (30 µg/cm²). Du milieu DMEM additionné de SVF à 10 % et de SAB à 2 % a été utilisé comme chimioattractant. Après une période d'incubation de 48 heures, les cellules ont été fixées avec du méthanol et colorées avec du violet cristal pendant 15 min. Les cellules restant sur la surface supérieure des membranes ont été éliminées par grattage et celles sur la surface inférieure ont été comptées en utilisant un microscope inversé (Pasco et al. 2000(a)). Les cellules comptées correspondent aux cellules qui ont traversé le tapis de Matrigel, puis la membrane poreuse de la chambre de Boyden ; elles sont donc le reflet des capacités de migration et d'invasion des cellules tumorales au travers d'une matrice extracellulaire ou d'une membrane basale, ici reproduite par le Matrigel.

A.11. Analyse de zymographie

Zymographie en présence de gélatine

L'expression de MMP-2 et MMP-9 dans un milieu conditionné de cellules UACC-903, traitées ou non avec des peptides synthétiques a été analysée par zymographie en présence de gélatine comme décrit précédemment (Pasco et al. 2000(a)). La sécrétion de TIMP-2 a été analysée dans un milieu conditionné de cellules UACC-903 par zymographie inverse en présence de gélatine comme décrit précédemment (Pasco et al. 2000(a)).

Zymographie en présence de gélatine et de plasminogène

Pour la détermination des activateurs du plasminogène, des milieux conditionnés de cellules UACC-903 ont été analysés par électrophorèse en gel de polyacrylamide en présence de SDS et contenant de la gélatine à 1 mg/ml et du plasminogène à 10 µg/ml. Après électrophorèse, les gels ont été incubés pendant une nuit à température ambiante dans un tampon glycine à 100 mM, EDTA à 5 mM pH 8.0. L'activité gélatinolytique résultant de l'activation du plasminogène a été mise en évidence par des zones de lyse blanches après coloration du gel avec du bleu de Coomassie (Pasco et al. 2004).

A.12. Western Blot

Les échantillons ont subi une électrophorèse dans un gel de polyacrylamide 10 % en présence de SDS à 0,1 %, puis transférés sur des membranes Immobilon-P (Millipore, St Quentin en Yvelines, France). Les membranes ont été saturées avec du lait écrémé lyophilisé à 5 %, Tween 20 à 0,1 % dans un tampon Tris-HCl à 50 mM, NaCl à 150 mM, pH 7,5 (TBS) pendant 2 heures à température ambiante. Les membranes ont ensuite été incubées pendant la nuit à 4° C avec des anticorps anti-MMP-14, anti-PAI-1 et anti-uPAR, puis incubées pendant une heure à température ambiante avec un anticorps secondaire (anti-IgG) conjugué à la péroxydase. Les complexes immuns ont été visualisés avec un kit de détection de chimioluminescence ECL.

A.13. Mesure de la croissance tumorale *in vivo*

Une suspension de cellules B16F1 (2.5 10⁵ cellules dans du milieu RPMI 1640) prétraitées ou non avec le cyclopeptide YSNSG (20 µM) a été injectée de façon sous-cutanée dans le flanc gauche de souris syngéniques de différentes séries. Les souris ont été sacrifiées au jour 20 et la taille des tumeurs a été mesurée. Le volume des tumeurs a été déterminé par la relation V = 1/2 A x B² dans laquelle A et B représentent respectivement la plus grande dimension et la plus petite dimension de la tumeur (Wald et al.).

A.14. Analyses statistiques

Les analyses statistiques ont été réalisées par le test t de Student. Les résultats sont exprimés comme la moyenne ± 1 écart type. Pour les expériences *in vivo,* les volumes des tumeurs primaires ont été analysés de façon statistique en utilisant le test -u non paramétrique de Mann et Withney et le test paramétrique t de Student, comparé avec des souris sélectionnées pour un poids correspondant.

A.15. Mesure de l'effet antiangiogénique

L'activité anti-angiogénique *in vitro* du cyclopeptide YSNSG est mesurée sur la formation de pseudotubes capillaires par les cellules endothéliales HMEC-1 ou HUVEC, déposées sur tapis de Matrigel.

Une solution de Matrigel (ECM gel : 10 mg/mL) est déposée stérilement dans des plaques à 24 puits (Nunclon), à raison de 250 µL par puits, sur de la glace pilée. La formation du gel est obtenue par incubation de la plaque à 37° C pendant 30 min. Des cellules HMEC-1 ou des cellules HUVEC à subconfluence sont détachées de leur support plastique de culture par addition de trypsine/EDTA, puis mises en suspension dans le milieu de culture pour cellules endothéliales et diluées à la concentration de 2.10⁵ cellules par mL. Des volumes de 100 µL de cette suspension cellulaire sont déposés à la surface du gel. Après incubation pendant 1 heure à 37° C pour permettre l'adhésion des cellules, les différents peptides sont ajoutés en solution dans 400 µL du milieu de culture (concentration finale 10 ou 20 µM). La formation de pseudotubes est observée sous microscope et photographiée, après 24 heures d'incubation. La quantification est réalisée avec un logiciel d'analyse d'images.

A.16. Technique de blessure cicatricielle

1.10⁵ cellules endothéliales sont ensemencées en plaques 24 puits et incubées 24 heures à 37°C. Une blessure est alors réalisée dans la monocouche cellulaire, au centre du puits, à l'aide d'une pointe de cône de 200 µl. Les cellules sont lavées avec du PBS pour éliminer les débris cellulaires, puis incubées en présence ou en absence de peptide cyclique YSNSG. La fermeture de la blessure, témoignant des propriétés migratoires des cellules, est observée en microscopie et photographiée après 48 heures d'incubation.

A.17. Coloration cytochimique au Hoechst-33342

Les cellules sont cultivées en plaque 24 puits, en présence ou non de cyclopeptide YSNSG. Après 24 heures d'incubation, les cellules sont rincées deux fois avec une solution de PBS. A cette étape, on ajoute sur les cellules du fluorochrome Hoechst-33342 (50 µg/ml) dilué dans du PBS. Après 15 minutes d'incubation à 37°C, 5% de CO₂, les noyaux des cellules sont observés à l'aide d'un microscope à fluorescence (λ excitation = 346 nm ; λ émission = 460 nm), puis photographiés.

A.18. Marquage de l'actine F

Les cellules sont ensemencées sur lamelles de verre stériles placées en plaque 24 puits (2.10⁴ cellules/puits), dans le milieu adéquat contenant 5 % de sérum, en présence ou non du cyclopeptide YSNSG. Après 24 et 48 heures d'incubation, les cellules sont fixées au formaldéhyde 3,7 % pendant 10 minutes à température ambiante et perméabilisées 3 minutes avec une solution d'acétone à -20 °C. Trois lavages de 5 minutes dans du PBS sont effectués, puis les cellules sont incubées 30 minutes à température ambiante dans une solution de saturation (PBS contenant 1 % de sérum albumine bovine). Les cellules sont alors incubées 1 heure avec la phalloïdine couplée à l'Alexa Fluor® 568 (diluée au 1/40 dans du PBS contenant 1 % de SAB) puis lavées 3 fois 5 minutes avec du PBS. Les lamelles sont observées avec un microscope confocal et photographiées.

A.19. Immunomarquage phospho-FAK et intégrine ß1

Les cellules sont ensemencées sur lamelles de verre stériles placées en plaque 24 puits (2.10⁴ cellules/puits), dans le milieu adéquat contenant 5 % de sérum, en présence ou non de cyclopeptide YSNSG. Après 24 et 48 heures d'incubation, les cellules sont fixées 15 minutes dans du méthanol. Trois lavages de 5 minutes dans du TBS-T sont effectués, puis les cellules sont incubées 30 minutes à température ambiante dans une solution de saturation (TBS-T contenant 3 % de SAB). Les cellules sont alors incubées 2 heures, à température ambiante, en présence de l'anticorps primaire dilué au 1/100 dans du TBS-T contenant 3 % de SAB. Après 5 lavages de 5 minutes en TBS-T, les cellules sont incubées avec l'anticorps secondaire correspondant couplé à l'Alexa Fluor® 488, dilué au 1/1000 dans du TBS-T contenant 3 % de sérum albumine bovine. Les cellules sont alors lavées 3 fois 5 minutes avec du TBS-T. Les lamelles sont observées avec un microscope confocal et photographiées.

**B. Résultats**

B.1. Structure du peptide linéaire YSNSG

Comme point de départ de l'étude du peptide linéaire, une simulation dynamique moléculaire a été réalisée à 300 K sur le peptide YSNSG linéaire avec une représentation explicite du solvant et en partant d'une conformation complètement étendue (φ = 180°; ψ = 180°). La simulation a duré pendant 20 ns. Cette expérience a permis de mettre en évidence dix groupes sur l'ensemble de la trajectoire, représentatifs de différentes structures rapportées à la Figure 2. Parmi ces groupes, on peut noter la présence de coudes β YSNS (groupes 1, 3 et 5), de coudes β SNSG (groupe 2), de structures intermédiaires (groupes 4 et 6) et de structures étendues ou quasi-étendues (groupes 7,8,9 et 10). De par leurs paramètres φ et ψ, les coudes β YSNS et SNSG sont très proches de coudes β de type I (résidus centraux des coudes localisés au niveau de la région de l'hélice α du diagramme de Ramachandran). Au vu de ces résultats et du maintien de la structure en coude β malgré l'addition d'un résidu glycine, il a donc été envisagé de produire un cyclopeptide YSNSG.

B.2. Structure du cyclopeptide YSNSG

La structure du cyclopeptide YSNSG a été étudiée tout d'abord par résonance magnétique nucléaire (RMN). Après l'attribution de tous les signaux protoniques sur le spectre TOCSY, l'une des informations les plus intéressantes provenait du spectre ROESY qui indiquait clairement une corrélation entre les protons amides du squelette de résidus séquentiels. Cependant, aucune corrélation n'était observée avec des résidus non séquentiels (Figure 3A). Quelques autres nOes ont été aussi révélés par les différents spectres dans les régions Nh-Hα (Figure 3B).

Une étude conformationnelle aléatoire de la structure du peptide a été réalisée en accord avec les données RMN (voir matériel et méthodes). De façon intéressante, les trois tests indépendants ont convergé vers une conformation identique de très faible énergie avec un RMSD de tous les atomes inférieur à 0,2 Å.

Cette structure (Figure 4) partage un coude β sur les résidus YSNS, avec une distance Cα(Y₁)-Cα(S₄) inférieure à 7,0 Å. La structure *in vacuo* a été stabilisée par des liaisons hydrogène entre les protons amides de Y₁ et la structure C=O de S₄ formant un coude γ sur des résidus S₄GY₁ et par des liaisons hydrogène entre le groupe hydroxyl de Y₁ et la fonction amide de la chaîne latérale de N₃.

Cette conformation a, par la suite, été utilisée comme point de départ d'une simulation dynamique moléculaire non contrainte qui a été réalisée dans les mêmes conditions que celle effectuée pour le peptide linéaire (eau explicite, 300 K). Comme attendu, le cyclopeptide a été observé sous la forme d'une structure hautement contrainte comme indiqué par la valeur RMSD inférieure à 0,5 Å sur les atomes du squelette, tout au long de la trajectoire donnée (non montré). Le coude γ décrit ci-dessus disparaît rapidement (durant la phase de chauffage) pour atteindre une conformation stable dans les premiers moments de la simulation. La flexibilité du peptide a ensuite été réduite pour explorer les différents rotamères de la tyrosine. La conformation correspondante a été caractérisée par le coude β YSNS pendant toute la trajectoire (Figure 5). Le diagramme des différents angles du squelette φ/ψ du peptide tout au long de la trajectoire a également montré que ces cinq résidus étaient souvent localisés dans une région hélice α du diagramme de Ramachandran, faisant du tétrapeptide YSNS une structure proche du coude β de type I. Cependant, ce coude n'appartient à aucun coude β de type canonique avec une moyenne φ/ψ de (- 90 °C ; - 66° C) pour à la fois les résidus S₂ et N₃.

Cette conformation est très proche du groupe 5 décrit pour le peptide linéaire avec un RMSD moyen d'environ 1,5 Å calculé pour les atomes du squelette.

B.3. Comparaison avec les données expérimentales

Pour comparer plus en détail les résultats expérimentaux et théoriques, un diagramme des distances moyennes proton/proton a été réalisé (représenté à la figure 6). Les distances moyennes sont extraites des simulations dynamiques moléculaires. A travers cette figure, on se rend compte que les nOes observés expérimentalement, particulièrement entre les protons amides des résidus séquentiels, correspondent à des distances moyennes inférieures à 4 Å. Au contraire, les protons amides du squelette qui ne se font pas face correspondent à des distances supérieures à 4 Å. De plus, la valeur moyenne observée des angles dièdres des deux résidus sérines était d'environ - 90°C en accord avec l'expérience RMN.

Un aspect particulier de la conformation du peptide, observé durant la trajectoire dynamique moléculaire, est que toutes les fonctions NH du squelette sont regroupées sur la même face du peptide alors que toutes les fonctions carbonyl sont regroupées sur l'autre face (Figure 7).

Cette conformation particulière empêche la formation de liaison hydrogène interne au squelette. Ce résultat était en accord avec les données expérimentales de la RMN. En effet, les coefficients de température des protons amides du peptide, qui ont été enregistrés entre 295 et 320 K, n'ont prédit aucune liaison hydrogène interne, avec des valeurs de - 10,0, - 4,6, - 7,0, - 4,6 et -6,1 ppb/K pour Y₁, S₂, N₃, S₄ et G₅ respectivement (des valeurs inférieures à - 4,5 ppb/K étant souvent associées à l'absence de liaison hydrogène). Du fait de cette caractéristique, le peptide apparaît hautement polarisé comme représenté à la figure 6 qui rapporte le potentiel électrostatique du peptide (isovaleurs de 7,5 et - 7,5 kT/e en position supérieure et inférieure respectivement). La distribution des charges a été calculée en utilisant les paramètres par défaut de la méthode « Particle Mesh Ewald » (Essmann et al.) telle que mise en oeuvre par VMD (Humphrey et al.). De façon intéressante, les charges positives englobant les chaînes latérales des résidus SNS sont connues pour jouer un rôle crucial dans l'activité biologique du peptide et de ses dérivés, caractéristique qui peut également être importante pour la fixation du peptide à l'intégrine αVβ3 (Pasco et al. ; 2000(b)).

B.4. Caractérisation de la structure secondaire du cyclopeptide YSNSG par spectroscopie dichroïsme circulaire

La figure 8 montre le spectre de dichroïsme circulaire (CD) du cyclopeptide YSNSG enregistré à pH 7 et à des températures de 0, 20, 37 et 50° C. Le signal obtenu ne montre pas clairement la présence d'une conformation bien structurée qui n'est ni totalement aléatoire, ni α ni β. Les mêmes résultats sont observés sans ajouter de tampon à un pH d'environ 3 ; ceci montre que le peptide est peu sensible à la température et au pH, dû probablement à son aspect hautement contraint.

La forme CD observée n'est caractéristique d'aucune conformation en coude β classique (type I, type II ou type VIII) ; ceci est probablement dû aux paramètres distordus φ/ψ du peptide comparé à un coude β de type I.

Puisque la RMN, le modèle moléculaire et les résultats CD ont mis en évidence que le cyclopeptide YSNSG adoptait la conformation attendue en coude β, son activité biologique a été comparée avec celle du peptide linéaire CNYYSNS.

B.5. Inhibition de la prolifération *in vitro* de cellules de mélanome humain UACC-903 par le cyclopeptide YSNSG

La prolifération *in vitro* des cellules de mélanome humain UACC-903 est inhibée de façon significative par l'heptapeptide linéaire CNYYSNS (-4.5%). Le cyclopeptide YSNSG à des concentrations de 5,10 et 20 µM inhibe aussi la prolifération des cellules de mélanome UACC-903 de 27, 29 et 40 % respectivement (Figure 9). Le cyclopeptide est approximativement aussi actif que le peptide naturel aux mêmes concentrations.

B.6. Inhibition de la migration *in vitro* de cellules de mélanome UACC-903 par le cyclopeptide YSNSG

L'effet du cyclopeptide YSNSG sur la migration des cellules UACC-903 sur une membrane basale reconstituée *in vitro* a été analysé en utilisant des membranes tapissées de Matrigel et du SVF à 10 % comme chimioattractant. La migration a été mesurée après 48 heures. Le cyclopeptide YSNSG induit une importante diminution de la migration des cellules UACC-903 (-47%), de façon similaire à l'heptapeptide linéaire CNYYSNS à une même concentration (Figure 10).

B.7. Effets du cyclopeptide YSNSG sur les métalloprotéinases matricielles et leurs inhibiteurs

Aucun changement significatif de la sécrétion de MMP-2 et MMP-9 n'a été détecté par zymographie en présence de gélatine après traitement des cellules UACC-903 par le cyclopeptide YSNSG ou le peptide linéaire CNYYSNS (Figure 11). Cependant, le cyclopeptide YSNSG inhibe fortement l'expression de proMMP-14 comme démontré par l'analyse de Western Blot (Figure 12). L'activation de proMMP-14 est également complètement abolie. Les effets inhibiteurs du cyclopeptide ont la même intensité que celle observée avec le peptide naturel.

La sécrétion des TIMP dans un milieu de culture conditionné a été analysée par zymographie inverse (Figure 13A). Une augmentation dose-dépendante de la sécrétion de TIMP-2 a été observée dans des cultures incubées avec le cyclopeptide YSNSG et également avec le peptide linéaire CNYYSNS (Figure 13B). Ce résultat a été confirmé en utilisant le système ELISA Biotrak (Amersham Biosciences, Orsay, France) (données non montrées). La sécrétion de TIMP-1 et TIMP-3 n'est pas modifiée.

B.8. Effets du cyclopeptide YSNSG sur le système d'activation du plasminogène

La sécrétion de u-PA et t-PA, les deux principaux activateurs du plasminogène, a été analysée par zymographie en présence de gélatine et de plasminogène (Figure 14). Le traitement des cellules UACC-903 avec le cyclopeptide YSNSG induit une diminution dose-dépendante de la sécrétion de u-PA et t-PA, plus forte que la diminution observée avec l'heptapeptide linéaire CNYYSNS.

La sécrétion de PAI-1, un inhibiteur de u-PA et t-PA, a été analysée par Western blot (Figure 15). Le traitement des cellules UACC-903 avec le cyclopeptide YSNSG induit une importante augmentation de la sécrétion de PAI-1, supérieure à celle observée avec l'heptapeptide CNYYSNS.

B.9. Inhibition de la croissance tumorale *in vivo* par le cyclopeptide YSNSG

L'ensemble des résultats *in vitro* indique que le cyclopeptide YSNSG inhibe la dégradation de la matrice extracellulaire et, dans le même temps, l'invasion des cellules tumorales de façon aussi efficace que le peptide naturel linéaire CNYYSNS. Par conséquent, une étude de ses effets *in vivo* a été réalisée.

Les effets du cyclopeptide YSNSG sur la croissance tumorale *in vivo* ont été étudiés dans un modèle murin de mélanome. Des cellules de mélanome murin B16F1, pré-incubées avec le cyclopeptide YSNSG ou non, ont été injectées de façon sous-cutanée dans le flanc gauche de souris syngéniques C57BL6 et le volume tumoral mesuré au jour 20. Les cellules B16F1 non traitées ont induit le développement de tumeurs sous-cutanées. La pré-incubation de cellules tumorales avec le cylopeptide YSNSG a inhibé la croissance tumorale de façon aussi efficace que l'heptapeptide linéaire CNYYSNS (Figure 16).

De façon complémentaire, une semaine après l'injection sous-cutanée de cellules de mélanome B16F1 dans le flanc gauche des souris C57BI6, à raison de 250000 cellules dans 0,1 mL de milieu RPMI 1640, les cyclopeptides selon l'invention sont administrés, dans diverses séries d'animaux, selon :
a. injection péritonéale de cyclopeptides (à une concentration de 5mg/kg de poids de souris) quotidiennement, seuls ou fixés sur des nanoparticules biodégradables ;
b. injection intraveineuse de cyclopeptides dans les mêmes conditions que l'option a) ;
c. administration *per* os : les cyclopeptides sont mélangés à de la nourriture (à raison de 10 mg/kg de poids de souris) et l'ensemble est administré par gavage gastrique ;
d. injection sous-cutanée de solution de cyclopeptides, controlatérale par rapport à l'injection des cellules cancéreuses ;

Dans tous les cas, l'effet des peptides est évalué par la mesure en cinétique du volume tumoral (pourcentage du volume tumoral avec traitement par rapport au volume tumoral sans traitement).

B.10. Effet anti-angiogénique du cyclopeptide YSNSG

L'activité anti-angiogénique est évaluée par la capacité des cellules endothéliales (cellules HMEC-1) à former des pseudo-tubes capillaires lorsqu'elles sont cultivées sur un gel de Matrigel. Comme le montre les photographies de la Figure 17A, l'addition de cyclopeptides (à 10 ou 20 µM) inhibe fortement la formation de pseudotubes de façon comparable à l'addition du peptide linéaire CNYYSNS. Ainsi, l'addition du cyclopeptide YSNSG à 10 µM réduit la formation de pseudo-tubes de 40%, tandis que l'addition du cyclopeptide YSNSG à 20 µM réduit la formation jusqu'à 70%, de façon similaire au peptide linéaire (Figure 17B).

Des résultats similaires ont été obtenus avec des cellules HUVEC, sur lesquelles le cyclopeptide YSNSG (20 µM) exerce une inhibition de 45%. Cette inhibition peut théoriquement s'exercer sur la prolifération cellulaire ou la migration cellulaire. Le cyclopeptide YSNSG inhibe de 83 % la migration des cellules HUVEC (Figure 18) après 48 h d'incubation dans un modèle de blessure artificielle. En revanche, le cyclopeptide YSNSG n'exerce aucune modification de la prolifération cellulaire.

Cette inhibition de la migration s'explique notamment d'une part par une diminution de 56 % de l'activation de la MT1-MMP, une métalloprotéinase matricielle capable de dégrader la matrice extracellulaire et largement impliquée dans l'angiogenèse (Figure 19), et d'autre part par une diminution de la production d'u-PA (-27 %), un activateur du plasminogène et de son récepteur, u-PAR, (-49 %), tous deux impliqués également dans la dégradation de la matrice extracellulaire et la migration cellulaire (Figures 20 et 21).

L'inhibition de la migration se manifeste par une modification de l'organisation du cytosquelette d'actine et par une diminution d'expression de la forme phosphorylée de la kinase d'adhésion focale (phospho-FAK) (Figure 22), témoignant d'une diminution du phénotype migratoire.

L'expression de la sous-unité β₁ d'intégrine en périphérie cellulaire est augmentée en présence du cyclopeptide (Figure 23), ce qui reflète une augmentation des points d'ancrage cellulaire et une diminution des propriétés migratoires des cellules.

**Discussion**

L'activité biologique du cyclopentapeptide YSNSG a été comparée à celle du peptide naturel CNYYSNS. Le cyclopeptide inhibe la prolifération *in vitro* des cellules de mélanome humain UACC 903, ainsi que leurs propriétés invasives évaluées par leur capacité de migration au travers de membranes recouvertes d'une matrice extracellulaire reconstituée, le Matrigel (Figures 9 et 10). L'intensité de l'inhibition obtenue est au moins égale à celle du peptide naturel, aux concentrations testées. Cette inhibition des propriétés invasives *in vitro* s'exerce par une très importante diminution de l'expression, mais surtout de l'activation de la MMP-14 (Figure 12), associée à une augmentation de la sécrétion d'inhibiteur TIMP-2 (Figure 13). Cette inhibition s'exerce également sur la cascade d'activation du plasminogène, par diminution de la sécrétion d'uPA et tPA, aussi intense que celle induite par le peptide naturel CNYYSNS (Figure 14).

De même, l'activité anti-cancéreuse du cyclopentapeptide YSNSG a été mesurée dans le modèle expérimental de mélanome *in vivo* chez la souris. Pour cela, des cellules B16F1 de mélanome de souris ont été traitées avec le cyclopentapeptide, puis injectées dans le flanc gauche de souris syngéniques C57BL6. Le cyclopentapeptide YSNSG induit une diminution du volume tumoral de 46% (Figure 16). Dans le cas présent, seul le traitement préalable des cellules a été utilisé, sans réinjection du peptide dans la région péri-tumorale aux jours 7 et 14.

### BIBLIOGRAPHIE

Brooks, B.R., Bruccoleri, R.E., Olafson, B.D., States, D.J., Swaminathan, S., & Karplus, M. (1983) J. Comp. Chem. 4,187-217.
Deryugina EI, Ratnikov B, Monosov E, Postnova TI, DiScipio R, Smith JW, et al. (2001) Exp. Cell Res., 263, 209-233.
Egeblad M, Werb Z. (2002) New functions for the matrix metalloproteinases in cancer progression. Nat. Rev. Cancer, 2, 161-173.
Essmann, U., Perera, L., Berkowitz, M. L., Darden, T., Lee, H., & Pedersen, L. G. (1995) J. Chem. Phys. 103, 8577.
Floquet N., Pasco S., Ramont L., Derreumaux P., Laronze J.Y., Nuzillard J.M., Maquart F.X. Alix A.J., Monboisse J.C. (2004) J. Biol Chem. 279(3): 2091-2100.
Han J, Ohno N, Pasco S, Monboisse JC, Borel JP, Kefalides NA. (1997) J. Biol. Chem., 272, 20395-20401.
Hornebeck W, Emonard H, Monboisse JC, Bellon G. (2002) Sem. Cancer Biol., 439, 1-11.
Humphrey, W., Dalke, A., & Schulten, K. (1996) J. Mol. Graph. 14, 33-38, 27-38.
Hutchinson EG, Thornton JM (1994) Protein Sci. Dec;3(12):2207-16
Kalé, L., Skeel, R., Bhandarkar, M., Brunner, R., Gursoy, A., Krawetz, N., Phillips, J., Shinozaki, A., Varadarajan, K. & Schulten, K. (1999) Journal of Computational Physics. 151, 283-312.
Kalluri R. (2003) Nat. Rev. Cancer, 3,422-433.
Kelley, L.A., Gardner, S.P. & Sutcliffe, M.J. (1996) Protein Eng. 9, 1063-1065.
MacKerell, J., A. D., Bashford, D., Bellott, M., Dunbrack, R. L., Jr., Evanseck, J. D., Field, M. J., Fischer, S., Gao, J., Guo, H., Ha, S., et al. (1998) Journal of Physical Chemistry B 102, 3586-3616.
Maeshima V; Sudhakar A, Lively JC, Ueki K, Kharbanda S, Kahn CR, Sonenberg N, Hynes RO, Kalluri R. (2002) 295, 140-143.
Maquart FX; Pasco S, Ramont L, Hornebeck W; Monboisse JC. (2004) Crit. Rev. Oncol. Haematol. 49, 199-202.
Martinella-Catusse C, Polelfe M, Noel A, Gilles C, Dehan P, Munaut C, et al. (2001) Lab. Invest. 81, 167-175.
Ortega N, Werb Z. (2002) J. Cell Sci. 11, 4201-4214.
Pasco S (2000a), Han J, Gillery P, Bellon G, Maquart FX, Borel JP, et al. Cancer Res. 60, 467-473.
Pasco S (2000b), Monboisse JC, Kieffer N. J. Biol. Chem. 275, 32999-33007.
Pasco S, Ramont L, Maquart FX, Monboisse JC. (2004) Crit. Rev. Oncol. Haematol. 49, 221-233.
Pasco S, Brassart B, Ramont L, Maquart FX, Monboisse JC. (2005) Cancer Detect Prev. 29, 260-266.
Pardi, A., Billeter, M., Jr., & Wuthrich, K. (1984) J. Mol. Biol. 180, 741-751.
Phillips, J.C., Braun, R., Wang, W., Gumbart, J., Tajkhorshid, E., Villa, E., Chipot, C., Skeel, R.D., Kale, L. & Schulten, K. (2005) J. Comp. Chem. 26, 1781-1802.
Piotto M., Saudek V and Sklenar V. (1992) J. Biomol. NMR, 1992, 2, 661.
Settor REB. (1998) Am. J. Pathol. 153, 1347-1351.
Shahan S, Ohno N, Pasco S, Borel JP, Monboisse JC, Kefalides NA (1999)Connect. Tissue Res. 40, 221-232.
Shahan TA, Ziaie Z, Pasco S, Fawzi A, Bellon G, Monboisse JC, et al. (1999) Cancer Res. 59, 4584-4590.
Thern B, Rudolph J, Jung G. (2002) Angew Chem Int Ed Engl. Jul 2;41 (13):2307-9
Wald, M., Olejar, T., Sebkova, V., Zadinova, M., Boubelik, M., & Pouckova, P. (2001) Cancer. Chemother. Pharmacol. 47, S16-S22.

## Revendications

1. Cyclopeptide choisi parmi :
a) un cyclopeptide homodétique, **caractérisé en ce qu'**il comprend la séquence YSNS; et
b) un cyclopeptide, dont le cycle comprend la séquence YSNS et a un nombre de résidus d'acides aminés compris entre 4 et 6.

2. Cyclopeptide selon la revendication 1 **caractérisé en ce que** le cycle est constitué par la séquence YSNS.

3. Cyclopeptide selon la revendication 1 ou 2, **caractérisé en ce que** sa taille est supérieure ou égale à 4 acides aminés et inférieure à 10 acides aminés, de préférence **en ce que** sa taille est de 4 à 6 acides aminés.

4. Cyclopeptide selon l'une quelconque des revendications 1 à 3, **caractérisé en ce qu'**il est capable de se fixer à l'intégrine αVβ3.

5. Cyclopeptide selon l'une quelconque des revendications 1 à 4 **caractérisé en ce qu'**il forme un coude β au niveau des acides aminés YSNS.

6. Cyclopeptide selon l'une quelconque des revendications 1 à 5, **caractérisé en ce que** sa séquence d'acides aminés consiste en YSNS.

7. Cyclopentapeptide homodétique consistant en la séquence YSNSX et représenté par la formule suivante : 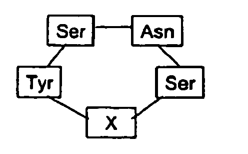 dans laquelle X est n'importe quel acide aminé permettant la cyclisation du peptide linéaire de même séquence.

8. Cyclopeptide selon la revendication 7, dans lequel X est un acide aminé de faible volume et/ou est un acide aminé faiblement chargé ou neutre.

9. Cyclopeptide selon l'une quelconque des revendications 7 à 8, **caractérisé en ce qu'**il est capable de se fixer à l'intégrine αVβ3.

10. Cyclopentapeptide dont la séquence d'acides aminés consiste en YSNSG, représentée par la formule suivante :

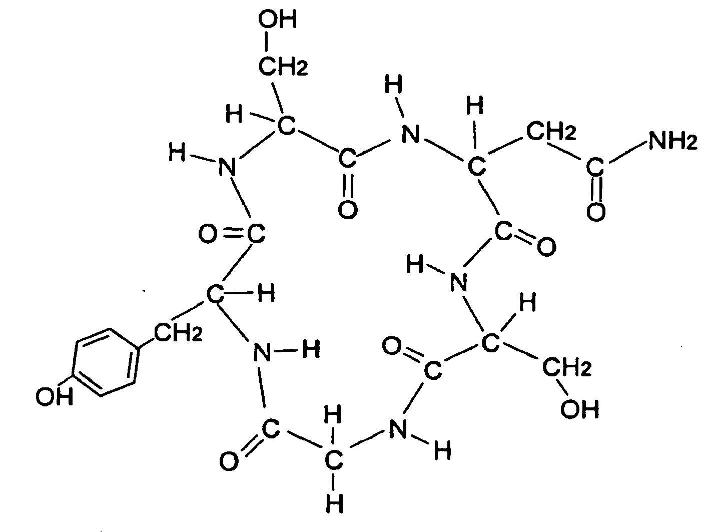

11. Cyclopeptide selon l'une quelconque des revendications 7 à 10, **caractérisé en ce que** toutes les liaisons peptidiques sont en trans, et optionnellement **en ce qu'**il a la structure tridimensionnelle représentée à la Figure 4.

12. Cyclopeptide selon l'une quelconque des revendications 1 à 11, **caractérisé en ce qu'**il possède des propriétés anti-tumorales.

13. Composition **caractérisée en ce qu'**elle comprend un cyclopeptide selon l'une quelconque des revendications 1 à 12, et optionnellement une molécule biologiquement active dans le traitement du cancer.

14. Composition selon la revendication 13, **caractérisée en ce que** la molécule biologiquement active est choisie parmi au moins une des molécules suivantes :
a. un agent chimiothérapeutique ;
b. un épitope tumoral spécifiquement associé aux cellules tumorales ;
c. un antigène de différenciation cellulaire ; et
d. l'interleukine 2 et/ou l'interféron α.

15. Cyclopeptide selon l'une quelconque des revendications 1 à 12, ou composition selon l'une quelconque des revendications 13 à 14 pour utilisation comme médicament.

16. Cyclopeptide selon l'une quelconque des revendications 1 à 12, ou composition selon l'une quelconque des revendications 13 à 14 pour utilisation dans le traitement du cancer, en particulier dans le traitement du mélanome ou dans le traitement du cancer bronchique, du cancer du sein ou du cancer de la prostate, ou en particulier dans le traitement un cancer dont les cellules tumorales expriment la molécule intégrine αVβ3.

17. Cyclopeptide selon l'une quelconque des revendications 1 à 12, ou composition selon l'une quelconque des revendications 13 à 14 pour utilisation dans l'inhibition ou la réduction de l'angiogenèse, de préférence au sein de tumeurs.

18. Cyclopeptide selon l'une quelconque des revendications 1 à 12, ou composition selon l'une quelconque des revendications 13 à 14 pour utilisation dans la diminution de la cascade protéolytique associée à proMMP-2 ou du système d'activation du *plasminogène (uPA).

19. Kit qui comprend une composition selon l'une quelconque des revendications 13 à 14, en particulier formulée pour une administration parentérale, orale, sous-cutanée ou intraveineuse.

## Claims

1. A cyclopeptide selected among:
a) a homodetic cyclopeptide **characterized in that** it comprises the sequence YSNS; and
b) a cyclopeptide whose cycle comprises the sequence YSNS and has a number of amino acid residues from 4 to 6.

2. A cyclopeptide according to claim 1, **characterized in that** the cycle is constituted by the sequence YSNS.

3. A cyclopeptide according to claim 1 or 2, **characterized in that** it is 4 or more amino acids and less than 10 amino acids in size; preferably, it is 4 to 6 amino acids in size.

4. A cyclopeptide according to any one of claims 1 to 3, **characterized in that** it is capable of binding to αVβ3 integrin.

5. A cyclopeptide according to any one of claims 1 to 4, **characterized in that** it forms a β-bend at the amino acids YSNS.

6. A cyclopeptide according to any one of claims 1 to 5, **characterized in that** its amino acid sequence consists of YSNS.

7. A homodetic cyclopentapeptide consisting of the sequence YSNSX and represented by the following formula: 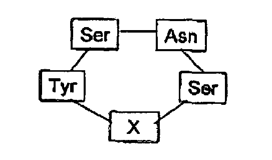 in which X is any amino acid that allows cyclisation of the same sequence-linear peptide.

8. A cyclopeptide according to claim 7, in which X is an amino acid with a small volume and/or a low charged or a neutral amino acid.

9. A cyclopeptide according to claim 7 or 8, **characterized in that** it is capable of binding αVβ3 integrin.

10. A cyclopentapeptide the amino acid sequence of which consists of YSNSG, represented by the following formula:

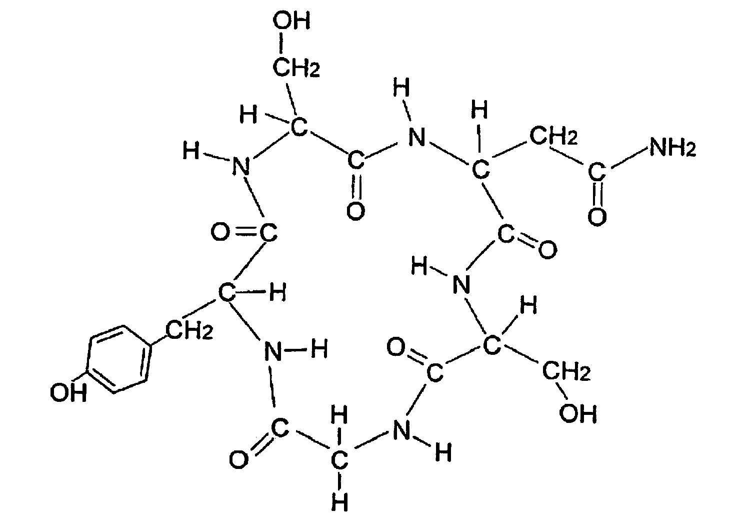

11. A cyclopeptide according to any one of claims 7 to 10, **characterized in that** all of the peptide bonds are in trans, and optionally **in that** it has the three-dimensional structure shown in Figure 4.

12. A cyclopeptide according to any one of claims 1 to 11, **characterized in that** it has anti-tumoral properties.

13. A composition **characterized in that** it comprises a cyclopeptide according to any one of claims 1 to 12, and optionally a molecule which is biologically active in the treatment of cancer.

14. A composition according to claim 13, **characterized in that** said biologically active molecule is selected from at least one of the following molecules:
a) a chemotherapeutic agent;
b) a tumoral epitope specifically associated with tumour cells;
c) an antigen for cellular differentiation; and
d) interleukin 2 and/or interferon α.

15. A cyclopeptide according to any one of claims 1 to 12, or a composition according to any one of claims 13 to 14, for use as a drug.

16. A cyclopeptide according to any one of claims 1 to 12, or a composition according to any one of claims 13 to 14, for use in the treatment of cancer, in particular in the treatment of melanoma or in the treatment of bronchial cancer, breast cancer or prostate cancer, or in particular in the treatment of cancer the tumour cells of which express the αVβ3 integrin molecule.

17. A cyclopeptide according to any one of claims 1 to 12, or a composition according to any one of claims 13 to 14, for use in inhibiting or reducing angiogenesis, preferably within tumours.

18. A cyclopeptide according to any one of claims 1 to 12, or a composition according to any one of claims 13 to 14, for use in reducing the proteolytic cascade associated with proMMP2 or the plasminogen activation system (u-PA).

19. A kit which comprises a composition according to any one of claims 13 to 14, in particular formulated for parenteral, oral, subcutaneous or intravenous administration.

## Patentansprüche

1. Cyclopeptid ausgewählt aus:
a) einem homodetischen Cyclopeptid, das **dadurch gekennzeichnet ist, dass** es die Sequenz YSNS umfasst, und
b) einem Cyclopeptid, dessen Ring die Sequenz YSNS umfasst und eine Anzahl an Aminosäureresten zwischen 4 und 6 aufweist.

2. Cyclopeptid nach Anspruch 1, **dadurch gekennzeichnet, dass** der Ring durch die Sequenz YSNS gebildet ist.

3. Cyclopeptid nach Anspruch 1 oder 2, **dadurch gekennzeichnet, dass** seine Größe größer oder gleich 4 Aminosäuren und kleiner als 10 Aminosäuren ist, bevorzugt **dadurch**, dass seine Größe von 4 bis 6 Aminosäuren beträgt.

4. Cyclopeptid nach einem der Ansprüche 1 bis 3, **dadurch gekennzeichnet, dass** es in der Lage ist, sich an das Integrin αVß3 zu binden.

5. Cyclopeptid nach einem der Ansprüche 1 bis 4, **dadurch gekennzeichnet, dass** es auf Höhe der Aminosäuren YSNS einen Knick ß bildet.

6. Cyclopeptid nach einem der Ansprüche 1 bis 5, **dadurch gekennzeichnet, dass** seine Aminosäuresequenz aus YSNS besteht.

7. Homodetisches Cyclopentapeptid bestehend aus der Sequenz YSNSX und dargestellt durch die folgende Formel: 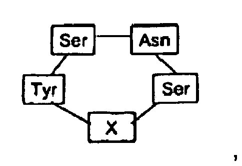 wobei X eine beliebige Aminosäure ist, die eine Cyclisierung des linearen Peptids mit derselben Sequenz ermöglicht.

8. Cyclopeptid nach Anspruch 7, bei dem X eine Aminosäure mit geringem Volumen und/oder eine gering geladene oder neutrale Aminosäure ist.

9. Cyclopeptid nach einem der Ansprüche 7 bis 8, **dadurch gekennzeichnet, dass** es in der Lage ist, sich an das Integrin αVß3 zu binden.

10. Cyclopentapeptid, dessen Aminosäuresequenz aus YSNSG besteht, dargestellt durch die folgende Formel:

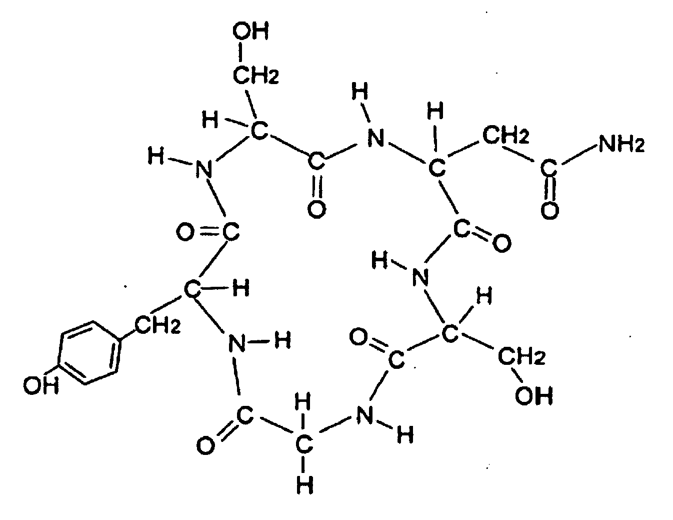

11. Cyclopeptid nach einem der Ansprüche 7 bis 10, **dadurch gekennzeichnet, dass** alle Peptidbindungen in trans vorliegen, und optional **dadurch**, dass es die in Figur 4 dargestellte dreidimensionale Struktur aufweist.

12. Cyclopeptid nach einem der Ansprüche 1 bis 11, **dadurch gekennzeichnet, dass** es anti-tumorale Eigenschaften aufweist.

13. Zusammensetzung, **dadurch gekennzeichnet, dass** sie ein Cyclopeptid nach einem der Ansprüche 1 bis 12 und optional ein Molekül, das bei der Behandlung von Krebs biologisch aktiv ist, umfasst.

14. Zusammensetzung nach Anspruch 13, **dadurch gekennzeichnet, dass** das biologisch aktive Molekül aus mindestens einem der folgenden Moleküle ausgewählt ist:
a. einem chemotherapeutischen Mittel,
b. einem Tumorepitop, das spezifisch Tumorzellen zugeordnet ist,
c. einem Antigen der Zelldifferenzierung und
d. Interleukin 2 und/oder Interferon α.

15. Cyclopeptid nach einem der Ansprüche 1 bis 12 oder Zusammensetzung nach einem der Ansprüche 13 bis 14 zur Verwendung als Medikament.

16. Cyclopeptid nach einem der Ansprüche 1 bis 12 oder Zusammensetzung nach einem der Ansprüche 13 bis 14 zur Verwendung bei der Behandlung von Krebs, insbesondere bei der Behandlung von Melanomen oder bei der Behandlung von Bronchialkrebs, Brustkrebs oder Prostatakrebs, oder insbesondere bei der Behandlung eines Krebses, dessen Tumorzellen das Molekül Integrin αVß3 exprimieren.

17. Cyclopeptid nach einem der Ansprüche 1 bis 12 oder Zusammensetzung nach einem der Ansprüche 13 bis 14 zur Verwendung bei der Inhibierung oder Reduktion der Angiogenese, bevorzugt im Inneren von Tumoren.

18. Cyclopeptid nach einem der Ansprüche 1 bis 12 oder Zusammensetzung nach einem der Ansprüche 13 bis 14 zur Verwendung bei der Verminderung der proteolytischen Kaskade, die mit proMMP-2 in Zusammenhang steht, oder des Plasminogen-Aktivierungssystems (uPA).

19. Kit, das eine Zusammensetzung nach einem der Ansprüche 13 bis 14 umfasst, insbesondere in einer Formulierung für eine parenterale, orale, subkutane oder intravenöse Verabreichung.
